# EUROPEAN PATENT APPLICATION

(11) **EP 4 799 719 A2**
(43) Date of publication of application: **02.09.2026**
(21) Application number: 26170957.0
(22) Date of filing: 01.09.2021
(51) Int. Cl.: B01F 33/452

(54) **CELL CULTURE SOLUTION CONTAINER AND DISPENSER**

(30) Priority: 01.09.2020 US 202063073356 P; 30.08.2021 US 202163238720 P
(62) Document number(s) of the earlier application(s) in accordance with Art. 76 EPC: 21778642.5 / 4 208 290
(71) Applicant: Stoic Bio, Inc., San Diego, CA 92127 (US)
(72) Inventor: SHEEHAN, David, San Diego, 92127 (US); BECERRA, Francisco Javier Martinez, San Diego, 92127 (US); HIOB, Matti, 00300 Colombo 3 (LK); MOHAMEDALI, Zainab Shiraz, 00300 Colombo 3 (LK); SELVARAJ, Varshini, 00300 Colombo 3 (LK); JAGANATHAMANI, Ridesh, 00300 Colombo 3 (LK); DIAS, Weerathanthirige Sanchitha Heshan, 00300 Colombo 3 (LK); MOULDS, J. Andrew, Carlsbad, 92011 (US); LARSON, Eric, Carlsbad, 92011 (US); PREMARATNE, Lasith Ishan, 00300 Colombo 3 (LK)
(74) Representative: V.O.

(57) **Abstract**

A container having a first end, a second end, and a deformable middle portion and methods for manufacturing the container are disclosed. The container has a collapsed configuration and an expanded configuration. In some embodiments, the first and second ends are rigid. In some embodiments, the container is configured to store cell culture solution powder. Methods and systems (e.g., a dispenser) for creating culture solution using the container are also disclosed.

## Description

### Cross-Reference to Related Applications

This application claims benefit of U.S. Provisional Application No. 63/073,356, filed September 1, 2020, and U.S. Provisional Application No. 63/238,720, filed August 30, 2021, the contents of each are incorporated herein by reference in their entirety.

### Field of the Invention

This disclosure generally relates to cell culture solution formulations. More specifically, this disclosure relates to cell culture solution containers and dispensers.

### Background of the Invention

Cell therapy extends and saves lives. Cancer survival rates, for example, have significantly increased as cell therapy solutions advanced. A critical component in cell therapy solutions is the cell culture media used to grow cells. Traditionally, cell culture media is manufactured as a bulk liquid, packaged in bottles, bags, or large drums ranging from 100 mL to 200 L, and then shipped to cell therapy engineers, bioproduction managers, and scientists. This approach has several drawbacks. The existing containers may be bulky and space-inefficient, increasing shipping costs. Once at the point-of-use, engineers and scientists must store the bulky containers on-site, sometimes requiring large storage areas so that a variety of cell culture medias will be available on-demand. Engineers and scientists may need to add other components to the media and then resterilize because of potential contamination, adding manual steps to the process and causing variability to the cell culture media.

### Summary of the Invention

A container having a first end, a second end, and a deformable middle portion and methods for manufacturing the container are disclosed. The container has a collapsed configuration and an expanded configuration. In some embodiments, the first and second ends are rigid. In some embodiments, the container is configured to store cell culture solution powder. Methods and systems for creating culture solution using the container are also disclosed.

In some embodiments, a cell culture solution container comprises: a rigid first end; a rigid second end; a deformable middle portion attached to the first end and second end and enclosing, with the first and second ends, a region having a variable volume; cell culture solution powder in the region; an inlet fluidly coupled to the region; and an outlet fluidly coupled to the region, wherein the container comprises a collapsed configuration and an expanded configuration.

In some embodiments, a volume of the region is greater in the expanded configuration than in the collapsed configuration.

In some embodiments, in the expanded configuration, the volume of the region accommodates a cell culture solution of 500 mL, 5 L, or 2000 L.

In some embodiments, a distance between the rigid first end and the rigid second end is greater in the expanded configuration than in the collapsed configuration.

In some embodiments, in the collapsed configuration, the distance is 0 mm, and in the expanded configuration, the distance is 120 mm or 300 mm.

In some embodiments, in the expanded configuration, the container has a height-to-width ratio of greater than one half.

In some embodiments, the ratio is 1.2.

In some embodiments, the inlet is located on the rigid first end, and the outlet is located on the rigid second end.

In some embodiments, the container further comprises a second inlet located on the rigid first end.

In some embodiments, the first and second inlets are parallel.

In some embodiments, the first inlet is a water inlet and the second inlet is an air inlet.

In some embodiments, the rigid first end is rectangular with a width of 85 mm and a length of 85 mm.

In some embodiments, the rigid first end is circular.

In some embodiments, when the container is in the expanded configuration, the middle portion comprises a rectangular cross-section.

In some embodiments, when the container is in the expanded configuration, the middle portion comprises a circular cross-section with a diameter of 73 mm.

In some embodiments, the rigid second end comprises a tapered surface on an interior to the container, the tapered surface tapering down and toward a center of the rigid second end.

In some embodiments, at least one of the rigid first end and the rigid second end is opaque to a sterilization light.

In some embodiments, the rigid first and second ends comprise at least one of polyethylene, poly lactic acid (PLA), and poly carpolactone (PCL).

In some embodiments, the middle portion is a polyethylene bag or a polypropylene bag.

In some embodiments, the rigid first and second ends and the middle portion comprise a recyclable material.

In some embodiments, the container further comprises an adapter attached to the inlet.

In some embodiments, the adapter comprises at least one of a Luer Lock adapter and a PVT adapter.

In some embodiments, the container further comprises a stirrer positioned in the region and having a length of 10-20 mm.

In some embodiments, the stirrer comprises polytetrafluoroethylene (PTFE).

In some embodiments, the container further comprises a QR code located on the rigid first end associated with the cell culture solution powder.

In some embodiments, the rigid first and second ends comprise a tongue or a groove configured to, during insertion of the container into a dispenser, couple the container and the dispenser.

In some embodiments, when the container is in the expanded configuration, the middle portion is flexible.

In some embodiments, a surface of the rigid first end is configured to interface with a surface of the rigid second end.

In some embodiments, wherein the middle portion stores a powder sachet, the powder sachet containing the solution powder.

In some embodiments, the powder sachet is attached to the rigid first end.

In some embodiments, the container further comprises a barrier in the middle portion separating the solution powder from at least one of the rigid first end and the rigid second end.

In some embodiments, the container further comprises a seal between the rigid second end and the middle portion.

In some embodiments, the container further comprises a seal between the rigid first end and the middle portion.

In some embodiments, the rigid first and second ends are configured to lock the container in the collapsed configuration when the rigid first end is rotated at a first angle from the rigid second end relative to an orientation of the rigid first and second ends when the container is in the expanded configuration.

In some embodiments, the rigid first end comprises a notch configured to mate with an opening of the rigid second end and lock the container in the collapsed configuration.

In some embodiments, the rigid first end comprises an opening.

In some embodiments, the container further comprises a cover configured to occlude the opening.

In some embodiments, the container further comprises a filter having a pore size of 0.22-1 µm.

In some embodiments, the container further comprises a filter attached to the outlet.

In some embodiments, the outlet is mounted on a spring mechanism.

In some embodiments, the outlet is configured to attach to an evacuation tube.

In some embodiments, the evacuation tube comprises at least one of a Luer Lock fitting and a sealing nozzle.

In some embodiments, the container further comprises a filter attached to the evacuation tube.

In some embodiments, the evacuation tube is bendable or flexible.

In some embodiments, the container further comprises a pH sensor sensing a pH level associated with the cell culture solution powder.

In some embodiments, the container further comprises a dissolution sensor.

In some embodiments, the cell culture solution powder is pre-granulated by spray addition of a pH correcting agent.

In some embodiments, in the collapsed configuration, the middle portion is configured to be vacuum-sealed.

In some embodiments, the container further comprises a material occluding the outlet, wherein the material is configured to break in response to an applied pressure above a pressure threshold.

In some embodiments, the material occluding the outlet has an area of 1 cm².

In some embodiments, the material comprises low-density polyethylene.

In some embodiments, the container further comprises a filter integrated with the outlet.

In some embodiments, a method of creating culture solution comprises: receiving, at a dispenser, a first container in a collapsed configuration; expanding the first container to an expanded configuration; while the first container is expanding or the first container is in the expanded configuration: injecting water into the first container; driving a stirrer in the first container; and mixing, with the stirrer, a powder and the water in the first container to create the culture solution; and dispensing the culture solution into a second container.

In some embodiments, the first container is a cell culture solution container described herein.

In some embodiments, the method of creating culture solution further comprises receiving an input, wherein the input is a selection on a graphical user interface (GUI) of the dispenser, and the first container is expanded in response to receiving the input.

In some embodiments, the method of creating culture solution further comprises receiving an input, and the input is a QR code scan of a QR code of the first container, and the first container is expanded in response to receiving the input.

In some embodiments, the method of creating culture solution further comprises prior to dispensing the culture solution into the second container, removing a cover of the second container.

In some embodiments, the method of creating culture solution further comprises providing a cover to the second container after dispensing the culture solution into the second container.

In some embodiments, the method of creating culture solution further comprises sterilizing the dispenser.

In some embodiments, the method of creating culture solution further comprises injecting air into the first container.

In some embodiments, the method of creating culture solution further comprises providing a filter, wherein the culture solution is dispensed into the second container through the filter.

In some embodiments, the method of creating culture solution further comprises collapsing the first container from the expanded configuration to the collapsed configuration.

In some embodiments, the stirrer is driven magnetically.

In some embodiments, the first container is any of the above cell culture solution containers.

In some embodiments, the method of creating culture solution further comprises applying a pressure to a middle portion of the first container.

In some embodiments, the pressure is 5-12 psi.

In some embodiments, the method of creating culture solution further comprises applying a second pressure to a chamber of the dispenser, the second pressure different from the first pressure.

In some embodiments, the method of creating culture solution further comprises applying a baffle to support a middle portion of the first container.

In some embodiments, the baffle is applied while the first container is expanding or the first container is in the expanded configuration.

In some embodiments, the method of creating culture solution further comprises performing inline testing of the culture solution.

In some embodiments, the method of creating culture solution further comprises refrigerating the culture solution at -10-4 degrees Celsius.

In some embodiments, the method of creating culture solution further comprises: heating a tube coupled between the dispenser and a destination; and transporting, via the tube, the culture solution to a destination, wherein the tube is heated to a temperature of the destination at 30-40 degrees Celsius.

In some embodiments, a cell culture solution dispenser is configured to perform any of the above methods of creating culture solution.

In some embodiments, a method of manufacturing a cell culture solution container for storing culture solution powder comprises: providing a rigid first end; providing a rigid second end; providing a deformable middle portion; attaching the deformable middle portion to the first end and second end, the first end, second end, and middle portion enclosing a region having a variable volume; providing an inlet; fluidly coupling the inlet to the region; providing an outlet; fluidly coupling the outlet to the region; and loading cell culture solution powder in the region.

In some embodiments, loading the culture solution powder into the region comprises loading the culture solution powder through an opening of the rigid first end.

In some embodiments, the method of manufacturing further comprises creating a vacuum in the region.

In some embodiments, creating the vacuum in the region comprises removing air from the region through an opening of the rigid first end.

In some embodiments, the method of manufacturing further comprises providing a stirrer.

In some embodiments, the stirrer comprises PTFE.

In some embodiments, the method of manufacturing further comprises providing a barrier in the middle portion separating the cell culture solution powder from at least one of the rigid first end and the rigid second end.

In some embodiments, providing the barrier further comprises folding the middle portion to create the barrier.

In some embodiments, the rigid first end comprises an opening.

In some embodiments, the method of manufacturing further comprises providing a cover configured to occlude the opening.

In some embodiments, providing the inlet comprises locating the inlet on the rigid first end, and providing the outlet comprises locating the outlet on the rigid second end.

In some embodiments, the method of manufacturing further comprises providing a second inlet located on the rigid first end.

In some embodiments, the method of manufacturing further comprises positioning the first and second inlets so that they are parallel.

In some embodiments, the rigid first end is rectangular.

In some embodiments, the rigid first end is circular.

In some embodiments, when the container is in an expanded configuration, the middle portion comprises a rectangular cross-section.

In some embodiments, when the container is an expanded configuration, the middle portion comprises a circular cross-section.

In some embodiments, the rigid second end comprises a tapered surface on an interior to the container, the tapered surface tapering down and toward a center of the rigid second end.

In some embodiments, the rigid first and second ends comprise at least one of polyethylene, PLA, and PCL.

In some embodiments, the middle portion is a polyethylene bag or a polypropylene bag.

In some embodiments, the rigid first and second ends and the middle portion comprise a recyclable material.

In some embodiments, the method of manufacturing further comprises attaching an adapter to the inlet.

In some embodiments, the adapter comprises at least one of a Luer Lock adapter and a PVT adapter.

In some embodiments, the method of manufacturing further comprises locating a QR code associated with the cell culture solution powder on the rigid first end.

In some embodiments, providing the rigid first and second ends comprises forming a tongue or a groove on the rigid first and second ends configured to, during insertion of the container into a dispenser, couple the container and the dispenser.

In some embodiments, the middle portion comprises a flexible material.

In some embodiments, the rigid first end and rigid second end comprises interfacing features.

In some embodiments, loading cell culture solution powder in the region further comprises providing a powder sachet comprising the cell culture solution powder.

In some embodiments, the method of manufacturing further comprises attaching the powder sachet to the rigid first end.

In some embodiments, the method of manufacturing further comprises providing a seal between the rigid first end and the middle portion.

In some embodiments, the rigid first end comprises a notch configured to mate with an opening of the rigid second end and lock the container in the collapsed configuration.

In some embodiments, the method of manufacturing further comprises providing a filter having a pore size of 0.22-1 µm.

In some embodiments, the method of manufacturing further comprises attaching a filter to the outlet.

In some embodiments, the method of manufacturing further comprises mounting the outlet on a spring mechanism.

In some embodiments, the method of manufacturing further comprises attaching an evacuation tube to the outlet.

In some embodiments, the evacuation tube comprises at least one of a Luer Lock fitting and a sealing nozzle.

In some embodiments, the method of manufacturing further comprises attaching a filter attached to the evacuation tube.

In some embodiments, the evacuation tube is bendable or flexible.

In some embodiments, the method of manufacturing further comprises providing a pH sensor sensing a pH level associated with the cell culture solution powder.

In some embodiments, the method of manufacturing further comprises providing a dissolution sensor.

In some embodiments, the method of manufacturing further comprises pre-granulating the cell culture solution powder by spray addition of a pH correcting agent.

In some embodiments, the cell culture solution powder is loaded in the region prior to attaching the deformable middle portion to the first end and second end.

In some embodiments, the container is any of the above cell culture solution container.

In some embodiments, the container is a container of any of the above methods of creating culture solution.

In some embodiments, the method of manufacturing further comprises occluding the outlet with a material, wherein the material is configured to break in response to an applied pressure above a pressure threshold.

In some embodiments, the material occluding the outlet has an area of 1 cm².

In some embodiments, the material comprises low-density polyethylene.

In some embodiments, a dispenser for manufacturing a cell culture solution, comprising: a graphical user interface (GUI) for controlling the dispenser; slots for receiving a first container and expanding the first container; a second container access mechanism for opening and closing a second container, wherein the dispenser dispenses the cell culture solution to the second container; a second container handling mechanism for moving the second container toward and away a dispensing location of the cell culture solution; a filter providing mechanism for providing a filter to the first container; a magnetic field generator for magnetically driving a stirrer in the first container; and a baffle for supporting a middle portion of the first container.

In some embodiments, the dispenser further comprises a pump for applying a first pressure to a middle portion of the first container.

In some embodiments, the first pressure is 5-12 psi.

In some embodiments, the pump is further for applying a second pressure to a chamber of the dispenser, and the first pressure is different from the second pressure.

In some embodiments, the dispenser further comprises a second pump for applying a second pressure to a chamber of the dispenser.

In some embodiments, the baffle is for supporting a middle portion of the first container.

In some embodiments, the baffle is applied while the first container is expanding or the first container is in the expanded configuration.

In some embodiments, the dispenser is configured to perform inline testing of the cell culture solution.

In some embodiments, the dispenser is configured to refrigerate the culture culture solution at -10-4 degrees Celsius.

In some embodiments, the dispenser is configured to couple to a tube between the dispenser and a destination; and heat the tube to a temperature of the destination at 30-40 degrees Celsius.

In some embodiments, a method of creating culture solution comprises: expanding a first container to an expanded configuration; while the first container is expanding or the first container is in the expanded configuration, mixing, a powder and water in the first container to create the culture solution; and dispensing the culture solution.

In some embodiments, the method of creating culture solution further comprises applying a pressure to a middle portion of the first container.

In some embodiments, the pressure is 5-12 psi.

In some embodiments, the method of creating culture solution further comprises applying a second pressure to a chamber of the dispenser, the second pressure different from the first pressure.

In some embodiments, the method of creating culture solution further comprises applying a baffle to support a middle portion of the first container.

In some embodiments, the baffle is applied while the first container is expanding or the first container is in the expanded configuration.

In some embodiments, the method of creating culture solution further comprises performing inline testing of the culture solution.

In some embodiments, the method of creating culture solution further comprises refrigerating the culture solution at -10-4 degrees Celsius.

In some embodiments, the method of creating culture solution further comprises: heating a tube coupled between the dispenser and a destination; and transporting, via the tube, the culture solution to a destination, wherein the tube is heated to a temperature of the destination at 30-40 degrees Celsius.

### Brief Description of the Drawings

Figures 1A and 1B illustrate exemplary containers.
Figure 2 illustrates an exemplary dispenser.
Figures 3A-3D illustrate an exemplary dispenser.
Figure 4 illustrates an exemplary method of operating an exemplary dispenser.
Figure 5 illustrates an exemplary method of manufacturing an exemplary container.
Figures 6A-6K illustrate an exemplary dispenser.

### Detailed Description of Exemplary Embodiments

In the following description of embodiments, reference is made to the accompanying drawings which form a part hereof, and in which it is shown by way of illustration specific embodiments which can be practiced. It is to be understood that other embodiments can be used and structural changes can be made without departing from the scope of the disclosed embodiments.

Exemplary containers herein may advantageously decrease shipping costs associated with transporting cell culture solution, may advantageously increase density of a shipment, and may advantageously decrease resources needed to store the cell culture solution before use. In some embodiments, the container contains powdered solution or media and takes a reduced profile for shipment. When needed for use, the container can expand and the powder mixed with water to provide a desired volume of cell culture solution (e.g., cell culture media) on demand. For example, after retrieving the container from storage or a shipment, a user can insert a collapsed container into a dispenser, the dispenser expands the container with the powder, the dispenser adds water, and the dispenser mixes the water and powder to create a desired volume of cell culture solution. Some embodiments of dispensers herein provide sterilization light to sterilize the container prior to mixing. Furthermore, in some embodiments, the disclosed container includes recyclable or reusable material, reducing waste and decreasing environmental footprint.

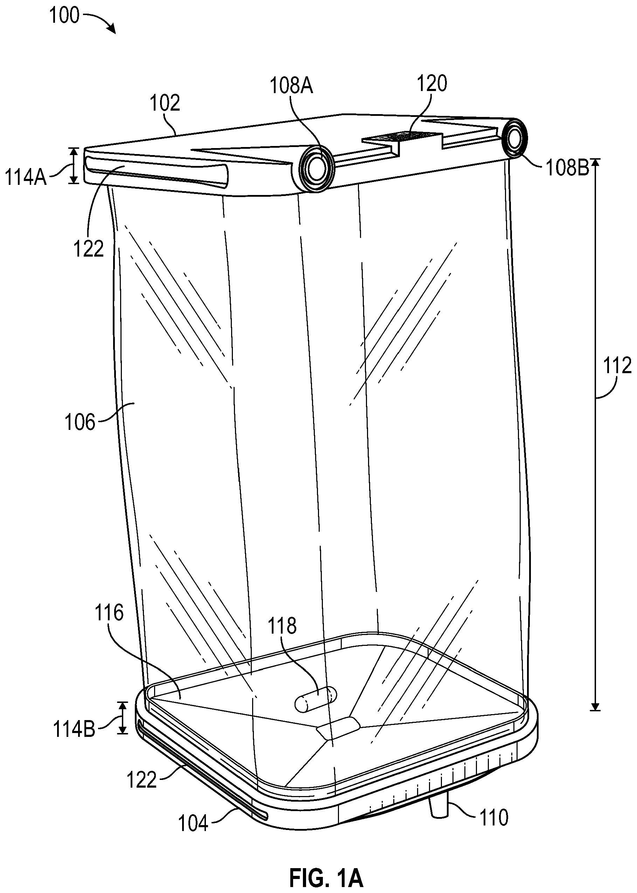

Figure 1A illustrates an exemplary container 100. In some embodiments, container 100 is a cell culture solution powder (e.g., cell culture media powder) container. Although the following discussion focuses on cell culture solution (e.g., cell culture media), it will be understood by those skilled in the art that container 100, container 150, dispenser 200, and dispenser 300 may be used for other applications. For example, although the disclosed examples are described with respect to cell culture solution (e.g., cell culture media), it is understood that container 100, container 150, dispenser 200, and dispenser 300 may be used more generally for cell growth media. As another example, it is understood that container 100, container 150, dispenser 200, and dispenser 300 may be also used in non-cell growth solution, such as salt solutions, dry buffer powder.

A person of ordinary skill in the art would recognize that for more general uses such as cell growth media or non-cell growth solution, requirements, steps, and elements may be adjusted accordingly for these uses. For example, those skilled in the art would recognize that requirements, steps, and elements associated with solubility and sterilization will be adjusted accordingly.

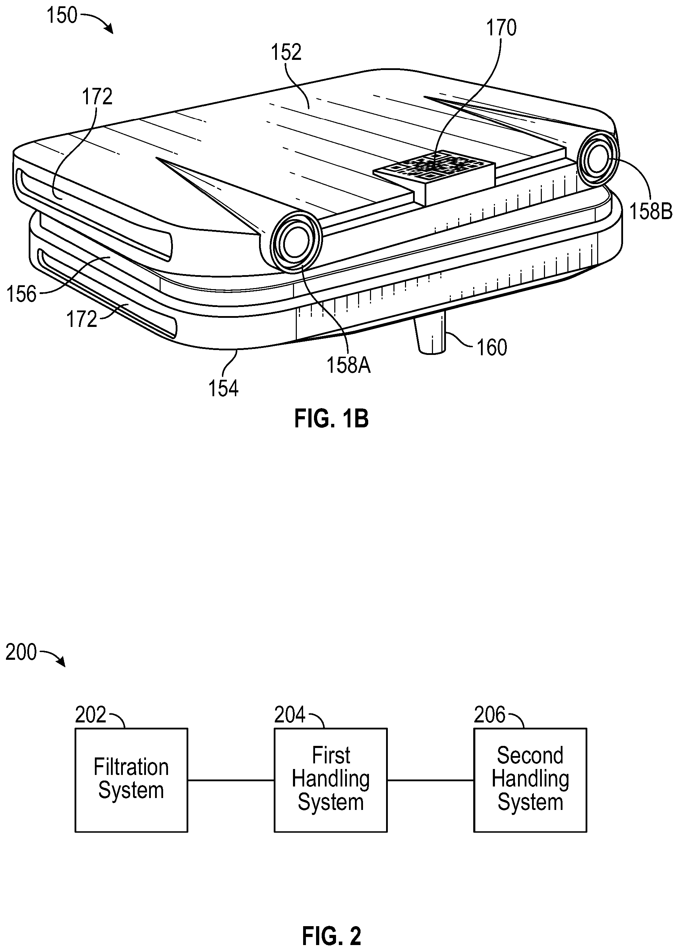

As illustrated, container 100 is in an expanded configuration and can be collapsible (e.g., to the configuration illustrated with respect to Figure 1B). Container 100 includes a first end 102, a second end 104, and a middle portion 106 attached to the first end 102 and to the second end 104. In some embodiments, the first end 102, the second end 104, and the middle portion 106 form a region 126 having a variable volume (e.g., the volume of the container may vary based on the configuration of the container 100). In some embodiments, the middle portion 106 is glued to the first end 102 and the second end 104, advantageously reducing components included in the container. In some embodiments, the first end 102 and the second end 104 are rigid.

In some embodiments herein, a container material can be understood to be rigid when it does not deform during shipping. Additionally, stiffness of rigid ends may cause a middle portion attached to the rigid ends to expand in response to a force pulling the rigid ends apart. In some embodiments, one or both of the rigid ends are opaque to block light from contacting the powdered media or solution inside and degrading any of the components, advantageously reducing additional resterilization steps and reducing variability to the cell culture solution (e.g., cell culture media). For example, existing containers are not opaque and sterilization of containers may adjust the chemical structure of the cell culture solution, thereby introducing variability.

As an exemplary advantage, a reduced profile may advantageously allow for more efficient shipping and reduce agitation of the powder during shipment. Further, rigid first and second ends may provide protection for the content to be stored in the container (e.g., during shipment when the container is in a collapsed configuration) because the content (e.g., cell culture solution powder) may be sensitive to contamination. As described and illustrated herein, the rigidity of the first and second ends allow the container to be expanded in response to an external force separating the ends while the rigid ends serve as an anchor to a dispenser coupled to the container, allowing the container to expand to a larger volume.

In some embodiments, the first end 102 has a thickness 114A, and the second end 104 has a thickness 114B. In some embodiments, the thickness 114A and the thickness 114B are each 10 mm. In some embodiments, the rigid first end and the rigid second end have rectangular cross-sections with radial corners, as illustrated in Figure 1. In some embodiments, the first end 102 and the second end 104 are rectangular. For example, when a desired volume of solution is 500 mL, the rectangular first and second ends have widths of 85 mm and lengths of 85 mm. As another example, when a desired volume of solution is 5 L, the rectangular first and second ends have widths of 170 mm and lengths of 170 mm. As another example, when a desired volume of solution is 500 L, the rectangular first and second ends have widths of 85 cm and lengths of 85 cm. As another example, when a desired volume of solution is 2000 L, the rectangular first and second ends have widths of 1.35 m and lengths of 1.35 m. In some embodiments, the rigid first end and the rigid second end are circular. In some embodiments, surfaces of the rigid first end and the rigid second end have a diameter of 73 mm.

Although specific values of quantities (e.g., dimensions, volumes, etc.) are provided, it is understood that in some embodiments, the corresponding values may not be exact. For example, a dimension or a volume may practically be a value functionally equivalent to the exact value. As an example, the value in these embodiments may be different from the exact value, as long as the difference is within a tolerance that does not functionally affect a system's accuracy, operation, and output.

Although the first end 102 and the second end 104 are described as having specific ranges of dimensions, it is understood that other dimensions may exist for the first and second ends without departing from the scope of the disclosure. Furthermore, it is understood that the described dimensions may not necessarily be uniform throughout the first end or the second end.

In some embodiments, the first end 102 and/or the second end 104 is opaque to a sterilization light. In some embodiments, the sterilization light is an ultraviolet light, gamma irradiation, or non-heat based radiation configured to sterilize components (e.g., components of the cell culture solution (e.g., cell culture media) dispenser, such as an air outlet and a water outlet) used in a cell culture solution (e.g., cell culture media) manufacturing process. As an exemplary advantage, by having container ends opaque to a sterilization light, the cell culture solution (e.g., cell culture media) powder container can be protected from the sterilization light (e.g., properties of the cell culture solution powder is prevented from being altered by the sterilization light) while component sterilization is being performed in a collapsed configuration.

In some embodiments, the second end 104 includes a surface 116 on an interior of the container that tapers down (e.g., away from a direction of the first end 102) and towards a center of the second end 104. For example, the rigid second end includes a tapered surface 116 with a minimum thickness at its center, as illustrated. As an exemplary advantage, the tapered surface 116 may allow a stirrer (e.g., a magnetic stir bar) to more efficiently mix a solution associated with the powder in the container by, for example, guiding the movements of the spinning stirrer as the solution is being mixed. The tapered surface 116 may also allow the container to drain more efficiently (e.g., the solution exits the container more efficiently). In some embodiments, a valve is attached to the outlet 110 (described in more detail herein) located on the second end 104; when the solution is being mixed, the valve is closed, preventing the solution from exiting the outlet 110. In some embodiments, when depressed under pressure, the valve is configured to allow fluid to flow. In some embodiments, prior to manufacturing of cell culture media, the valve separates the content from the filter, preventing the content from clogging the filter.

In some embodiments, the first end 102 and the second end 104 include at least one of polyethylene, PLA, and PCL. The material is advantageously included based on environmental needs of a user of the container. In some embodiments, the first end 102, the second end 104, and the middle portion 106 (described in more detail herein) include recyclable material(s). As an exemplary advantage, by using a cell culture solution (e.g., cell culture media) powder container including recyclable material, the environmental footprint of the cell culture solution manufacturing process may be reduced because less plastic may be used or a type of plastics having faster decomposition properties may be used (e.g., compared to PET bottles, compared to bottles that may be treated as biohazard waste) and waste may be minimized. In some examples, the container may be recycled (e.g., the same container may be returned to a manufacturer for resterilization and reuse).

In some embodiments, container 100 includes a stirrer 118 positioned in the region 126. In some embodiments, the stirrer has a length of 10-20 mm and is rectangular or cylindrical. In some embodiments, for bioproduction applications (e.g., the container accommodates a cell culture solution of 2000 L), the stirrer has a length of greater than 20 mm. The cylindrical stir bar may advantageously allow for more efficient mixing of the solution, compared to other stir bar geometries. Further, a cylindrical stir bar can advantageously reduce tearing of the middle portion during sealing, shipment, etc. In some embodiments, the stirrer includes PTFE (e.g., a PTFE based material). In some embodiments, the stirrer 118 includes a magnetic material and is configured to mix a solution associated with the cell culture solution (e.g., cell culture media) powder residing in the container. In some embodiments, the magnetic stir bar rotates (e.g., clockwise or counter-clockwise, relative to the surface 116) in response to an applied magnetic field (e.g., a time-varying magnetic field created by a cell culture dispenser that the container is coupled to) and mix a solution associated with the cell culture solution (e.g., cell culture media) powder residing in the container (e.g., mixing the powder and provided water). In some embodiments, the stir bar may alternate clockwise rotation and anti-clockwise rotation to improve mixing. In some embodiments, a center of the magnetic field is shifted from a center of the container to move a position of the stirrer away from the center of the second end.

In some embodiments, container 100 includes a QR code 120 attached to the first end 102 and associated with a content to be stored in the container. In some embodiments, the QR code 120 is attached to an access port cover 124, a hood, or a cap covering an opening (e.g., an access port) of the first end 102. Although the QR code 120 is described as being attached to the first end 102 or the access port cover 124, it is understood that the QR code 120 may be attached to second end 104, or the middle portion 106). For example, the QR code 120 may identify the cell culture solution (e.g., cell culture media) powder to be stored in the container and an associated formula for preparing a specific cell culture solution (e.g., cell culture media) formulation. A cell culture solution (e.g., cell culture media) dispenser or system may use the QR code (e.g., by using a scanner, by using a camera) to quickly identify the content of the container and the formula associated with the content and be configured (e.g., provide a suitable amount of water, provide a suitable amount of air, determine stirring time, determine a pH of the solution) to manufacture the cell culture solution (e.g., cell culture media) based on the content. In some embodiments, the dispenser is configured to communicate with a manufacturing facility to provide data on the dispenser's use. Although a QR code is used to describe the identification of a content to be stored in the container, it is understood that other elements (e.g., a bar code, a pattern, radio frequency identification, etc.) may identify the content to be stored without departing from the scope of the disclosure.

In some embodiments, the first end 102 and the second end 104 include guides 122 configured to couple the ends to a dispenser with complimentary ports for the inlet and the outlet (as depicted in Figure 1A, guides 122 are grooves that couple with complimentary tongues on a dispenser to guide the container during insertion). In some embodiments, container 100 includes another guide (not shown) located on the opposing end of the first end 102 from upper guide 122 illustrated in Figure 1A and another guide (now shown) on the opposing end of the second end 104 from lower guide 122 illustrated in Figure 1A. Advantageously, the guides 122 may be configured to allow the container to slide into a dispenser (e.g., a cell culture solution dispenser to manufacture cell culture solution). In some embodiments, the guides and the position of the inlets may advantageously limit installation of the container to a single orientation, allowing a user to quickly and efficiently insert the container into the machine. In some embodiments, guides 122 securely hold container 100 in place to allow a separation force to be applied to ends 102 and 104. In some embodiments, the guides 122 securely hold collapsed container 100 while a dispenser (described further below) applies a force to change container 100 to an expanded configuration. The guides 122 may also advantageously secure the container and its content to the dispenser during the mixing process. Features of the guides 122 are described elsewhere herein.

In some embodiments, container 100 includes an opening (e.g., an access port) located on the rigid first end. In some embodiments, the opening is a sealable opening located on the rigid first end configured to provide access to the region 126 for the addition of powder and to then seal and/or vacuum the region 126 after the powder is added. In some embodiments, the sealable opening is occluded by an access port cover 124, a sterile hood, or a cap including a material similar to the rigid first end.

In some embodiments, the middle portion 106 is deformable (e.g., to the configuration illustrated with respect to container 150; the middle portion is flexible, as described herein; the middle portion is foldable, as described herein). In some embodiments, the container includes cell culture solution (e.g., cell culture media) powder in the region 126.

In some embodiments, in the expanded configuration, the middle portion 106 of container 100 is expanded. In some embodiments, in the expanded configuration, a max volume of container 100 (e.g., when middle portion 106 is expanded) accommodates a specific volume of solution, where the volume is in the range of 500 mL to 2000 L. In some embodiments, in the expanded configuration, the first and second ends are separated by a distance 112. For example, the distance 112 is 120 mm when a volume of the container in an expanded configuration accommodates a 500 mL solution. As another example, the distance 112 is 300 mm when a volume of the container in an expanded configuration accommodates a 5 L solution. As another example, the distance 112 is 1.2 m when a volume of the container in an expanded configuration accommodates a 500 L solution. As another example, the distance 112 is 1.9 m when a volume of the container is in an expanded configuration for mixing a 2000 L solution.

By designing the middle portion 106 to have a distance 112, mixing and container size may be advantageously optimized. For example, for a container in an expanded configuration for mixing a 500 mL solution, the distance 112 is 120 mm (e.g., a width of the middle portion is 85 mm, and a length of the middle portion is 85 mm). The total volume of the exemplary container in the expanded configuration is 867 mL, allowing a level of the 500 mL solution to rise while mixing (e.g., the solution becomes vortex shaped such that a top surface of a solution rises higher, compared to a top surface of a still 500 mL solution) and allowing the middle portion to fit (e.g., the middle portion did not become too large, such that it would not fit within the first and second ends) within the first and second ends while the container is in a collapsed configuration.

The volume of the container in the expanded configuration may differ based on the application. For example, a container having an expanded configuration volume for mixing a 500 mL or a 5 L solution may be used in a laboratory environment to produce a smaller volume of cell culture solution (e.g., cell culture media), and a container having an expanded volume for mixing a 500 L or 2000 L solution may be used in a factory environment (e.g., bioprocessing) to produce a larger volume of cell culture solution (e.g., cell culture media).

In some embodiments, in the expanded configuration, the container has a height-to-width ratio greater than one half (e.g., the height of the container is greater than a width of the container). For example, the ratio is 1.2. As an exemplary advantage, a container having a height-to-width ratio greater than one half facilitates solubilization of content in the container (e.g., a distance from a stirrer to the content being mixed, across a plane of the second end, is reduced). For example, using a 15 mm stirrer and rotating the stirrer at 2000 RPM (e.g., to mix the container contents), content in a container having a height-to-width ratio greater than one half (e.g., 1.2) has a greater solubility (e.g., measured based on conductivity of the content). That is, the content converges toward an optimal solubility faster (e.g., around 400 seconds), compared to a container having a height-to-width ratio less than one half (e.g., around 800 seconds).

In some embodiments, a volume of the container is greater in the expanded configuration than the collapsed configuration. In some embodiments, a distance between the first end and second end is greater in the expanded configuration than the collapsed configuration. For example, in the expanded configuration, a distance between the first end and the second end is 120 mm, and a volume of the container is 867 mL. In the collapsed configuration, the distance between the first end and the second end is less than 120 mm (e.g., 0 mm) and the volume of the container is less than 500 mL (e.g., 20 mL).

In some embodiments, the middle portion 106 is flexible. For example, material of middle portion 106 stretches beyond its resting shape in response to a pulling force. In some embodiments, the middle portion 106 is not flexible. For example, material of the middle portion 106 does not stretch in response to a pulling force (for example, the middle portion is an accordion shape that allows for elongation of the middle portion when pulled, but the material does not stretch). In some embodiments, the middle portion 106 is deformable in response to an external force to alter the configuration of the container and mixing regime associated with content of the container.

In some embodiments, when the container is in the expanded configuration, the middle portion 106 includes a rectangular cross-section (as illustrated). For example, for a container having a volume for mixing a 500 mL solution at an expanded configuration, the rectangular middle portion has a width of 85 mm and length of 85 mm. As another example, for a container having a volume for mixing a 5 L solution at an expanded configuration, the rectangular middle portion has a width of 170 mm and length of 170 mm. In some embodiments, when the container is in the expanded configuration, the middle portion includes a circular cross-section (not shown). For example, the diameter of the circular cross-section is 73 mm.

Although the dimensions of container 100 is described with respect to a distance between two points, it is understood that the shape of the middle portion may not be perfectly cubic (e.g., an edge of the middle portion is not straight).

In some embodiments, middle portion 106 is a polyethylene bag, a polypropylene bag, or other sterilizable plastic bag attached to the first end 102 and the second end 104. In some embodiments, the middle portion 106 is a multi-layer bag including a polyethylene layer for heat sealing and nylon for reducing gas exchange. In some embodiments, a base of the bag is tucked into or clipped onto the second end 104. For example, the second end 104 includes features (e.g., the second end 104 includes a first piece and a second piece that hold the base of the bag together) that allow the base of the bag to snap into the second end 104. In some embodiments, the container includes a first seal (not shown) between the rigid first end and the middle portion, and the container includes a second seal (not shown) between the rigid second end and the middle portion. The seal may advantageously protect the contents of the container (e.g., protect the powder from contamination, protect the solution from contamination during mixing).

In some embodiments, the cell culture solution (e.g., cell culture media) powder is stored in a powder sachet (not shown). For example, the middle portion 106 is configured to store the powder sachet containing the powder. In some embodiments, the powder sachet includes a 20-50 µm mesh. In some embodiments, when the container is in an expanded configuration, the powder sachet is configured to be displaced from the rigid second end (e.g., attached to the rigid first end, attached to the middle portion).

In some embodiments, the container includes a barrier in the middle portion separating the powder from at least one of the first end and the second end. In some embodiments, the barrier is at a height from the rigid second end when the container is in the expanded configuration. In some embodiments, the barrier is located at a height selected to allow a predetermined volume of water to be added to the container before dissolution begins.

The powder sachet and/or the barrier may advantageously keep a stirrer in the middle portion away from the cell culture solution (e.g., cell culture media) powder prior to mixing or before enough water is added to the container during mixing. Before mixing or when sufficient water is added to the container during mixing, the powder sachet and/or the barrier may dissolve, dropping the powder onto the rigid second end and allowing mixing to begin.

In some embodiments, container 100 includes inlets 108A and 108B and an outlet 110. In some embodiments, an inlet 108A or 108B is located on the first end 102, and the outlet 110 is located on the second end 104. In some embodiments, the inlets 108A and 108B and outlet 110 are fluidly coupled to the region 126 of the container. In some embodiments, two elements are fluidly coupled if a fluid (e.g., water, air, a solution) can flow between the elements. For example, a fluid can flow between the inlets 108 and the region 126 within the container (e.g., water or air can follow from an inlet to the region 126), and a fluid can flow between the region 126 within the container and outlet 110 (e.g., a solution can flow from the region 126 to the outlet). In some embodiments, the outlet 110 has a height of 16 mm. In some embodiments, cell culture solution (e.g., cell culture media) powder and/or a stirrer are loaded into the container through one of the inlets or the outlet. In some examples, the cell culture solution (e.g., cell culture media) powder and/or a stirrer are loaded into the middle portion prior to assembling the container. In some embodiments, the content of the container is weighed and blended prior to being loaded into the container.

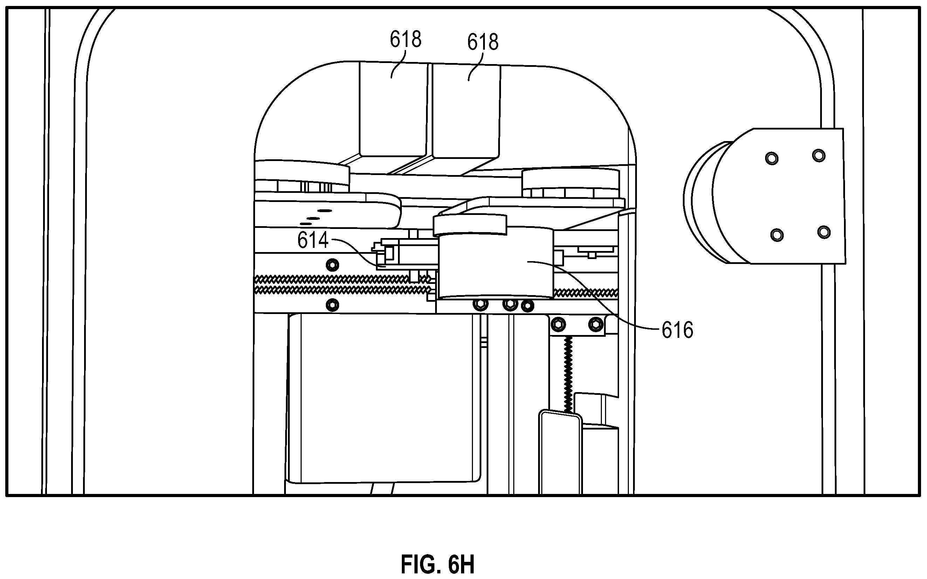

In some embodiments, the outlet 110 is located such that when a filter is coupled to the outlet, the coupled filter does not physically impede a magnetic field generator from aligning with the second end of the container while the magnetic field generator drives a stirrer during mixing. For example, as described with respect to Figure 6H, the outlet of the container 608 is located such that when a filter is coupled to the outlet (by the filter coupling mechanism 614), the coupled filter does not physically impede the magnetic field generator 616.

In some embodiments, a material occludes the outlet 110. In some embodiments, the material occluding the outlet is different than the material of the surface 116. In some embodiments, the material occluding the outlet comprises low-density polyethylene. In some embodiments, the material occluding the outlet is configured to tear or break open in response to an applied pressure (e.g., 5-10 psi) above a pressure threshold (e.g., 2-5 psi). In some embodiments, the material occluding the outlet has an area of 1cm². The material occluding the outlet may advantageously keep the content (e.g., cell culture solution powder) of the container 100 sterile until the content is being used for manufacturing a solution. During manufacturing, when a solution is ready to exit the container, a pressure above the pressure threshold is applied in the container (e.g., by using pumping air, by collapsing the container 100), causing the material to tear or break and allowing the solution to exit the container 100 through the outlet 110. In some embodiments, prior to manufacturing of cell culture media, the material occluding the outlet separates the content from the filter, preventing the content from clogging the filter.

In some embodiments, the container 100 has a larger volume (e.g., 500 L, 2000 L) and is used with a bioreactor. The cell culture solution (e.g., cell culture media) powder for containers having a larger volume may be loaded from bags (e.g., 20 L) and drums into the container through an opening of the container (e.g., an opening on the first end of the container).

In some embodiments, the inlets 108A and 108B are located on the first end 102. In some embodiments, the inlets 108A and 108B are located on a same side of the first end 102. In some embodiments, inlets 108A and 108B are parallel so that they connect to the corresponding attachments on a dispenser while container 100 is inserted into the dispenser. In some embodiments, one of the inlets 108A and 108B is an air inlet, and the other is a water inlet. In some embodiments, the container includes one inlet, and the inlet is an air and/or water inlet. In some embodiments, the air inlet is configured to couple to an air outlet of a cell culture solution (e.g., cell culture media) dispenser. In some embodiments, a HEPA filter is placed at the air inlet for sterilization by controller airflow. In some embodiments, the water inlet is configured to couple to a water outlet of a cell culture solution (e.g., cell culture media) dispenser. In some embodiments, the water inlet is configured to transport United States Pharmacopoeia (USP) grade water or highly purified water suitable for manufacturing cell culture solution (e.g., cell culture media) from a cell culture solution (e.g., cell culture media) dispenser to a middle portion of the container.

As an exemplary advantage, a dispenser (e.g., a cell culture solution (e.g., cell culture media) dispenser) configured to couple to the inlets and the outlet of the container may better align to the container because the fixed positioning of the inlets and the outlet reduces complexity of alignment between the container and the dispenser, even for different kinds of media powders.

In some embodiments, the container 100 includes an adapter (not shown) attached to an inlet 108A or 108B. In some embodiments, the adapter includes at least one of a Luer Lock adapter and a PVT adapter. As an exemplary advantage, the adapter allows for simple, but reliable interface between the container and a cell culture solution (e.g., cell culture media) dispenser.

In some embodiments, container 100 includes a filter (not shown). In some embodiments, the filter is a sterile filter. In some embodiments, the filter is a filter cartridge. During mixing, the filter may be used to filter undesirable by-product in the solution as the solution exits the container (e.g., through the outlet 110). In some embodiments, the filter has a pore size of 0.22 µm to 1 µm. In some embodiments, the filter is attached to the outlet 110 separately. In some embodiments, a filter is integrated with the outlet 110 (e.g., the filter is part of the container assembly, the filter is part of a base of the container, the filter is pre-attached to the outlet prior to manufacturing cell culture media, the filter is part of the outlet), advantageously reducing a need to separately attach the filter to the outlet.

In some embodiments, the filter is provided by a dispenser (e.g., dispenser 300, a cell culture solution (e.g., cell culture media) dispenser). For example, the filter is stored in the dispenser, and in response to an input (e.g., a mechanical input or electrical input from a user, a determination by the dispenser), the dispenser provides the filter. In some embodiments, the filter is installed onto the outlet 110 of the container (e.g., using the spring mechanism described). In some embodiments, the filter is installed onto the outlet 110 of the container manually. In some embodiments, the dispenser automatically installs the filter onto the container (e.g., before the solution exits the container, when the solution is ready to be transferred to another container for storage).

As an exemplary advantage, by providing the filter separately and installing the filter onto the container at a point of use, the size of the container may be reduced (e.g., to increase shipping efficiency), and the content of the container may be less likely damaged or contaminated. Furthermore, different types and sizes of filters may be provided, allowing a more suitable filter to be chosen for each use (e.g., including a suitable filter with the container may not be cost or size efficient).

In some embodiments, the outlet 110 is configured to attach to an evacuation tube (not shown). To optimize shipping volume, the evacuation tube may be attached after shipping and before mixing. In some embodiments, during or after mixing, the evacuation tube guides the solution to a second container for storing the solution. In some embodiments, the evacuation tube includes a Luer Lock fitting, sterilizable plastic tube, and a sealing nozzle. In some embodiments, the evacuation tube includes a filter used to filter undesirable by-product in the solution as the solution exits the container. In some embodiments, the evacuation tube is bendable or flexible. The bendable or flexible evacuation tube may allow the tube to be more efficiently shipped and allow the tube to be more easily guided to a second container for storing the solution during or after mixing.

In some embodiments, the outlet 110 is mounted on a spring mechanism. This allows the outlet to automatically close and open in response to the filter (e.g., provided by dispenser 300, provided by a cell culture solution (e.g., cell culture media) dispenser) being inserted or in response to an another force associated with dispensing the mixed solution.

In some embodiments, the container includes a pH sensor (not shown) sensing a pH level associated with a content to be stored in the container. A pH level of the media powder may be advantageously monitored during its lifetime (e.g., from being loaded into the container, to being shipped, to being mixed) to ensure product quality. In some embodiments, the media powder is pre-granulated by spray addition of a pH correcting agent (e.g., an acid, a base) at a determined quantity. For example, the pH correcting agent is added to the media powder on a fluid bed to facilitate drying and incorporation of the correcting agent with the powder. In some embodiments, the container includes a dissolution sensor (not shown) configured to determine a level of dissolution associated with the content of the container (e.g., if the solution is adequately mixed).

Although container 100 is described with respect to the elements described in Figure 1A, the container may include additional or less elements than expressly described, without departing from the scope of the disclosure. For example, although container 100 is illustrated as having two inlets, it is understood that other numbers of inlet may be included in the container without departing from the scope of the disclosure.

Figure 1B illustrates an exemplary container 150. In some embodiments, the container 150 is a cell culture solution (e.g., cell culture media) powder container. Although the container 150 is a cell culture solution (e.g., cell culture media) powder container in some embodiments, it is understood that the container 150 may be used for other applications. As illustrated, the container 150 is in a collapsed configuration and can be expandable (e.g., to the configuration illustrated with respect to Figure 1A). In some embodiments, in the collapsed configuration, the middle portion 156 is enclosed by the first end 152 and the second end 154, securing the content within the middle portion (e.g., media powder, stirrer, elements able to be stored in the container described with respect to Figure 1).

As an exemplary advantage, from shipment to formulation, the content (e.g., cell culture solution (e.g., cell culture media) powder) can reside in a same collapsible and expandable container and may not need to be transferred from one container to another, reducing risks of contaminating the content. As another exemplary advantage, the collapsibility of the container allows contents of the container to be easily expelled from the container (e.g., squeezing the contents out after usage) to allow the container to be more recyclable or be more ready for resterilization and be reused.

In some embodiments, the container changes from the expanded configuration the collapsed configuration or from the collapsed configuration to the expanded configuration using a tool or a machine. For example, when the container is in an expanded configuration (e.g., when content of the container is being loaded), the first and second ends of the container are slid into or secured to slots of the tool or the machine. The slots of the machines are apart from each other at a distance of a height of the container in the expanded configuration. The tool or the machine can change the container from the expanded configuration to the collapsed configuration (e.g., after the container is ready to be packaged, shipped, or stored) by decreasing the distance between the slots (e.g., a slot is controlled by a descending mechanism, reducing the distance between the slots).

In some embodiments, the container 150 includes similar elements described with respect to Figure 1A (e.g., the container 150 is the container 100, but in the collapsed configuration). As such, for brevity, the description of these similar elements will not be described again with respect to Figure 1B. For example, the first end 152 corresponds to the first end 102, the second end 154 corresponds to the second end 104, the middle portion 156 corresponds to the middle portion 106, the inlets 158A and 158B corresponds to the inlets 108A and 108B, the outlet 160 corresponds to the outlet 110, the QR code 170 corresponds to the QR code 120, and the guides 172 corresponds to the guides 122.

In some embodiments, in the collapsed configuration, the middle portion 156 of the container 150 is collapsed. In some embodiments, in the collapsed configuration, the first and second ends are in contact (e.g., when the container in the collapsed configuration, a distance between the first and second end is 0 mm). In some embodiments, the thickness of the container in the collapsed configuration is 20 mm. In some embodiments, a shipping thickness of the container (e.g,, the thickness of the container and the height of the outlet) in the collapsed configuration is 36 mm.

In some embodiments, the container 150 is configured to stack with another container. In some embodiments, a surface of the first end 152 is configured to interface with a surface of the second end 154. In some embodiments, a top surface of a first end 102 of a first container is configured to interface with a bottom surface of a second end 154 of a second container. In some embodiments, the top surface of the rigid first end and the bottom surface of the rigid second end is flat, allowing adjacent containers to be stacked. In some embodiments, the top surface of the rigid first end and the bottom surface of the rigid second end includes features (e.g., groves, guides, notches, inserts) that allow the two surfaces to interface securely, allowing a plurality of containers to be efficiently and securely packed for shipping and storage (e.g., compared to more bulky containers that are not space efficient). Therefore, using the disclosed containers to store cell media culture powder may advantageously decrease shipping costs associated with transporting the powder, increase density of a shipment, and decrease resources (e.g., storage space) needed to store the powder before use. In some embodiments, during shipment or storage, an outlet 160 of the container 150 is placed in a protective packaging to prevent the outlet (and its interface to a filter) from being damaged.

In some embodiments, to prevent contamination and to maximize the shelf life of the container content, the middle portion 156 of the container 150 is vacuum-sealed. For example, the first end includes an opening (e.g., an access port) and the opening is configured to allow the vacuum to be created (e.g., allow a tube to remove air from the region 126 of the container). In some embodiments, after air is removed from the middle portion, the middle portion is sealed (e.g., the opening allows access to a portion of the bag to be sealed) and the opening is covered by an access port cover 174, a hood, or a cap, securing the vacuum in the middle portion.

In some embodiments, a barrier is used to prevent contamination and to elongate shelf life of the container content during shipment and storage or separate between different contents in the container. In some embodiments, the barrier is formed by the middle portion 156 when the container is in the collapsed configuration. For example, the middle portion 156 includes a foldable bag. When the container is in the collapsed configuration, the bag is folded such that the folds of the bag serves as sealed barriers (e.g., the content is separated from at least one of the first end and the second end by the barrier) for the content in the barrier. In some embodiments, the sealed barriers in the middle portion 156 are configured to maintain the vacuum within the middle portion 156 when the container is in the collapsed configuration.

In some embodiments, the barrier of the middle portion 156 is created when the container changes from the expanded configuration to the collapsed configuration (e.g., when the container content is being loaded, after the content is loaded, after a first content is loaded and before a second content is loaded). In some embodiments, as the container changes (e.g., during a method of manufacturing a cell culture container, such as manufacturing methods described herein) from the expanded configuration to the collapsed configuration, a tool (e.g., an off-center slit that the middle portion passes through) causes the middle portion to fold in a specific manner, such that the fold of the middle portion (e.g., the fold of a bag) creates the barrier.

In some embodiments, the barrier of the middle portion 156 separates a first content and a second content in the container while the container is in the collapsed configuration. When the container changes from the collapsed configuration to an expanded configuration (e.g., before mixing of the content), the barrier opens and allows the first and second content to be together in the middle portion.

Although the container 150 is described with respect to the elements described in Figure 1B, the container may include additional or less elements than expressly described, without departing from the scope of the disclosure. For example, although the container 150 is illustrated as having two inlets, it is understood that other numbers of inlets may be included in the container without departing from the scope of the disclosure.

In some embodiments, the first end 152 and the second end 154 include guides 172 configured to couple the ends to a dispenser configured to couple to the inlet and the outlet. In some embodiments, the guides 172 are located on opposing ends of the first end 102 and on opposing ends of the second end 154, as illustrated. Advantageously, the guides 172 may be configured to allow the container to slide into a dispenser (e.g., a cell culture solution (e.g., cell culture media) dispenser coupling to the container to manufacture cell culture solution (e.g., cell culture media)) configured to couple to the container and the container to be securely installed into the dispenser.

In some embodiments, prior to manufacturing cell culture solution (e.g., cell culture media), the guides 172 slide into slots of a cell culture solution (e.g., cell culture media) dispenser. In some embodiments, the container is in a collapsed configuration when the container is installed (e.g., slide into slots of the cell culture solution (e.g., cell culture media) dispenser like a cassette tape) into the cell culture solution (e.g., cell culture media) dispenser. In some embodiments, after the container is securely installed into the dispenser, the guides 172 keep the rigid first and second ends at a fixed position relative to the slots that guides 172 are interfacing with. In some embodiments, with the guides 172 keeping the rigid first and second ends at a fixed position relative to the slots, the cell culture solution (e.g., cell culture media) dispenser causes the slots to part vertically, such that the middle portion 156 expands. As such, the containers 100 and 150 may advantageously allow the content to be efficiently shipped and adaptable to a machine for media formulation.

In some embodiments, the first end 152 and the second end 154 are configured to lock the container 150 in the collapsed configuration when the first end 102 is rotated at a first angle from the second end 154 relative to an orientation of the first end 102 and the second end 154 when the container is in the expanded configuration (e.g., twisted like a cap). In some embodiments, turning the first end 102 clockwise relative to the second end 154 locks the container 150 in the collapsed configuration, and turning the first end 102 counter-clockwise relative to the second end 154 unlocks the container 150 from the collapsed configuration (e.g., allowing the container to expand).

In some embodiments, the first end 152 includes a notch configured to mate with an opening of the second end 154 and lock the container in the collapsed configuration. In some embodiments, the first end 152 includes notches that can interface (e.g., mate, attach, couple) with corresponding openings on the second end 154 to lock the container in the collapse configuration. A user may pull the first end 152 and the second end 154 away from each other to detach the two rigid ends away from each other.

By allowing the first and second ends to lock when the container is in a collapsed configuration, the content of the container may be advantageously secure during shipment and/or storage. In some embodiments, the middle portion of the container is in a vacuum when the container is in the collapsed configuration. The vacuum in the middle portion may be advantageously maintained by allowing the container to lock.

Figure 2 illustrates an exemplary dispenser 200. In some embodiments, the dispenser 200 is a cell culture solution (e.g., cell culture media) dispenser. For example, the cell culture solution (e.g., cell culture media) dispenser is used with a smaller container (e.g., 500 mL, 5 L) (e.g., in a laboratory setting). As another example, the cell culture solution (e.g., cell culture media) dispenser is used with a larger container (e.g., 500L, 2000 L) (e.g., in a bioreactor). As illustrated, the dispenser 200 includes a filtration system 202, a first handling system 204, and a second handling system 206. In some embodiments, the dispenser 200 includes a first handling system 204 and a second handling system 206, and the filtration system 202 is included in a different dispenser.

In some embodiments, the filtration system 202 is an ultra-pure water (UPW) filtration system. For example, the UPW filtration system includes a water inlet configured to receive water (e.g., UPW water, USP water, water from an external source). In some embodiments, the water passes through a UPW filter of the filtration system and into a water reservoir of the filtration system 202. In some embodiments, the water circulates between the filter and the reservoir to maintain or increase the purity of the water. In some embodiments, the water in the filtration system is provided (e.g., pumped) to the first handling system 204.

In some embodiments, the first handling system 204 includes a container chamber, an inlet, container handling mechanism, and a mixing system. In some embodiments, the container chamber is an enclosure for insertion, expansion, and/or collapsing of a container (e.g., container 100, container 150, container 308, container 608). In some embodiments, the container chamber is sterilized (e.g., using a sterilization light). For example, prior to mixing container content, the container chamber is sterilized (e.g., by the dispenser 200, by a device different from the dispenser 200). In some embodiments, the dispenser 200 comprises a heating chamber to heat water and air to sterilize the dispenser's piping. In some embodiments, the dispenser 200 comprises an ozone generator for removing contaminants in the chamber (e.g., to purify water, to purify air).

In some embodiments, the inlet of the first handling system 204 is an inlet configured to couple to an inlet of a container (e.g., inlet 108A, inlet 108B, inlet 158A, inlet 158B). In some embodiments, the inlet provides water (e.g., from the filtration system 202) and/or air for the container. In some embodiments, the inlet of the first handling system 204 advantageously aligns with an inlet of a container when the container is inserted, allowing installation of the container into the dispenser to be more efficient. In some embodiments, the inlet is cleaned or sterilized (e.g., using a sterilization light). For example, prior to mixing container content, the inlet is cleaned or sterilized (e.g., by the dispenser 200, by a device different from the dispenser 200).

In some embodiments, the container handling mechanism includes an insertion mechanism (e.g., slots 304) configured to accept a container as the container (e.g., container 100, container 150, container 308, container 608) is being inserted (e.g., the slots are configured to match the guides (e.g., guides 122, guides 152) of the container) and secure the container after the insertion of the container. In some embodiments, the insertion mechanism includes two portions (e.g., slots 304A and 304B) that are configured to move apart. Because the container is secured to the insertion mechanism, when the container is in a collapsed configuration, as the insertion mechanism moves apart, the insertion mechanism causes the container to expand. In some embodiments, the insertion mechanism includes two portions (e.g., slots 304A and 304B) that are configured to move closer together. Because the container is secured to the insertion mechanism, when the container is in an expanded configuration, as the insertion mechanism moves closer together, the insertion mechanism causes the container to collapse.

In some embodiments, the mixing system includes hardware for driving a stirrer used for mixing the contents of a container. For example, the mixing system includes a magnetic field generator for driving a magnetic stirrer for mixing. As an example, the magnetic field generator generates a time-varying magnetic field to drive or guide the stirrer to rotate as intended to mix the contents of the container.

In some embodiments, the first handling system 204 provides a filter (e.g., a filter cartridge). For example, the filter is provided from the dispenser 200, and is loaded on a rail aligned with the container. The filter is coupled to an outlet of the container and the content of the container (e.g., cell culture solution (e.g., cell culture media)) passes through the filter before dispensing the content into a second container. In some embodiments, after the content is dispensed into the second container, the first handling system 204 disposes the used filter. In some embodiments, a filter is integrated with the outlet, advantageously reducing a need to separately attach the filter to the outlet and/or dispose the filter. As such, the first handling system 204 does not separate attach the filter to the outlet and/or separately dispose the filter.

In some embodiments, after the content is dispensed into the second container, the first handling system 204 disposes the used container. In some embodiments, the used container is ejected and is reused, as described herein.

In some embodiments, the second handling system 206 includes a second container chamber and a second container access mechanism. In some embodiments, the second container chamber is an enclosure for insertion of a second container (e.g., container 306, a media bottle) and dispensing of content from a first container (e.g., container 100, container 150, container 308, container 608) to the second container. In some embodiments, the container chamber is sterilized (e.g., using a sterilization light). For example, prior to dispensing the first container content, the second container chamber is sterilized (e.g., by the dispenser 200, by a device different from the dispenser 200).

In some embodiments, the second container access mechanism includes a motor driven element that is configured to unscrew a cap of the second container (e.g., in accordance with a determination that the solution in the container is ready for dispensing). In some embodiments, while the cap of the second container 306 is being removed, the second container is being lowered (e.g., by the second container access mechanism) to separate the cap from the second container. In some embodiments, the second container access mechanism includes a motor driven element that is configured to screw a cap of the second container (e.g., in accordance with a determination that the solution in the container has been completely transferred). In some embodiments, the motor driven element screwing and unscrewing the cap is a same element. In some embodiments, while the cap of the second container is installed, the second container is raised (e.g., by the second container access mechanism) to mate the cap with the second container. In some embodiments, an interface between the second container access mechanism and the cap of the second container is adjustable (e.g., the diameter of the interface is adjustable) to accommodate different container sizes.

In some embodiments, the dispenser 200 includes a user interface, hardware, and software for operating the dispenser. In some embodiments, the dispenser 200 includes a scanner (e.g., a QR code scanner) for scanning information on a container (e.g., scanning QR code 120, scanning QR code 170). Advantageously, instructions for operating the dispenser 200 may be updated through software and firmware updates.

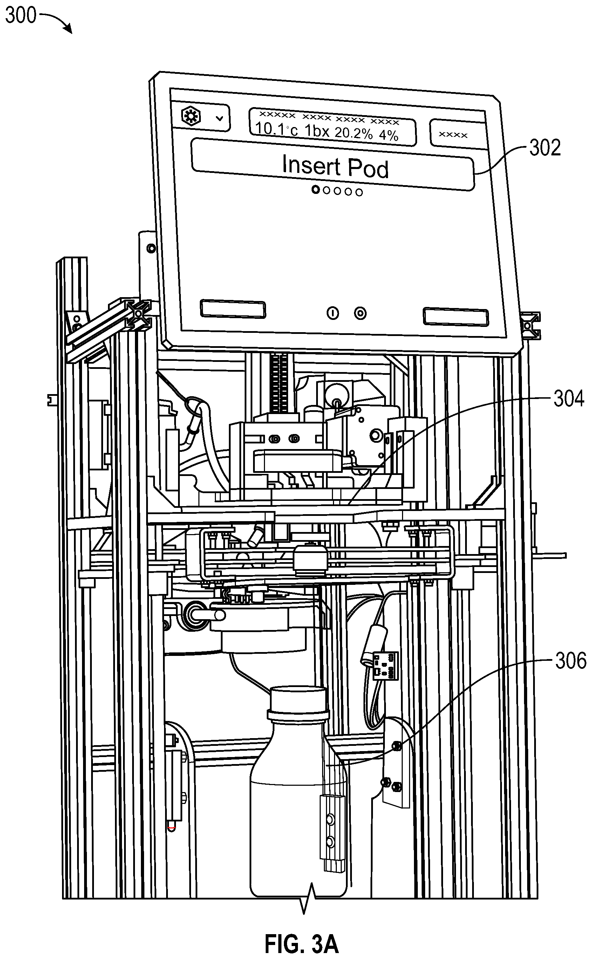

Figures 3A-3D illustrates an exemplary dispenser 300. In some embodiments, the exemplary dispenser 300 is dispenser 200. In some embodiments, the dispenser 300 is a cell culture solution (e.g., cell culture media) dispenser. Although the exemplary dispenser 300 is described with respect to a smaller container (e.g., 500 mL), it is understood that a larger container (e.g., 5 L,500L, 2000 L) may be used (e.g., expanded, its content being mixed) in a similar manner without departing from the scope of the disclosure. For example, the larger container may be used to create a larger volume solution for a bioreactor.

Figure 3A illustrates the dispenser 300 prior to installation of a cell culture solution (e.g., cell culture media) powder container (e.g., container 100, container 150). In some embodiments, the dispenser 300 includes a display configured to present a GUI 302 and slots 304. In some embodiments, the dispenser 300 is configured to dispense cell culture solution into the second container 306.

In some embodiments, the GUI 302 is displayed on a touch screen and is configured to receive input for cell culture solution (e.g., cell culture media) manufacturing. In some embodiments, prior to mixing, the GUI 302 displays a prompt for a user to install or insert a container (e.g., container 100, container 150) including media powder used for manufacturing cell culture solution (e.g., cell culture media). In some embodiments, the GUI 302 includes user-configurable options that defines aspects of the cell culture solution (e.g., cell culture media) manufacturing process.

In some embodiments, the slots 304 are configured to accept a container (e.g., container 100, container 150) including media powder used for manufacturing cell culture solution (e.g., cell culture media). In some embodiments, the guides (e.g., guides 122, guides 172) of the container are configured to interface with the slots such that the container can be slid into the slots using the guides. In some embodiments, when the container is completely slid into the slots, the slots 304 secures the container, preventing the container from moving (e.g., during mixing). In some embodiments, the dispenser 300 includes air and water outlets (not labeled) that can be coupled to inlets (e.g., inlets 108A and 108B, inlets 158A and 158B) when the container is sliding into the slots or otherwise before the content of the container is mixed. In some embodiments, the air and water outlets align with inlets (e.g., inlets 108A and 108B or 158A and 158B) such that sliding the container into the slots would cause the outlets of the dispenser to couple with the inlets of the container without additional adjustment to align the container with the dispenser. In some embodiments, the air and water outlets of the dispenser 300 provide air and water (e.g., USP grade water, water suitable for cell culture solution (e.g., cell culture media) manufacturing).

Although the slots 304 of the dispenser 300 are described to guide the container 308 during insertion and keep the container 308 secure during expansion, it is understood that insertion of the container may be guided or the container may be kept secured during expansion using other components. For example, a component with different mechanical features (e.g., allowing the container to snap to the dispenser), with electromagnetic features, or with adhesive properties may be used along with or instead of the slots 304.

In some embodiments, the second container 306 stores a solution (e.g., a media bottle) associated with the content of the installed or inserted container 308 (e.g., a solution produced by the dispenser 300). In some embodiments, the solution exits an outlet (e.g., outlet 110, outlet 160) of a container before being transferred to the second container 306.

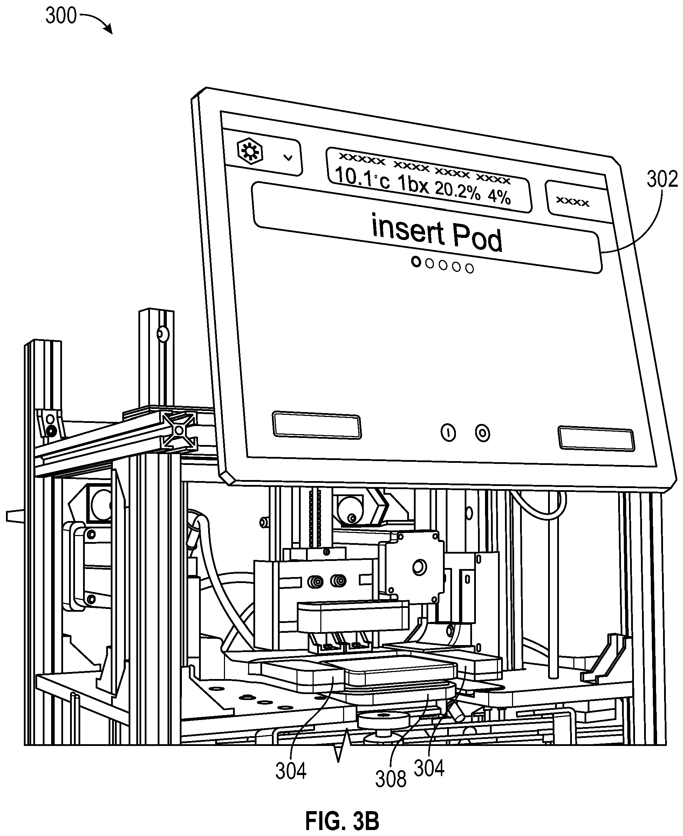

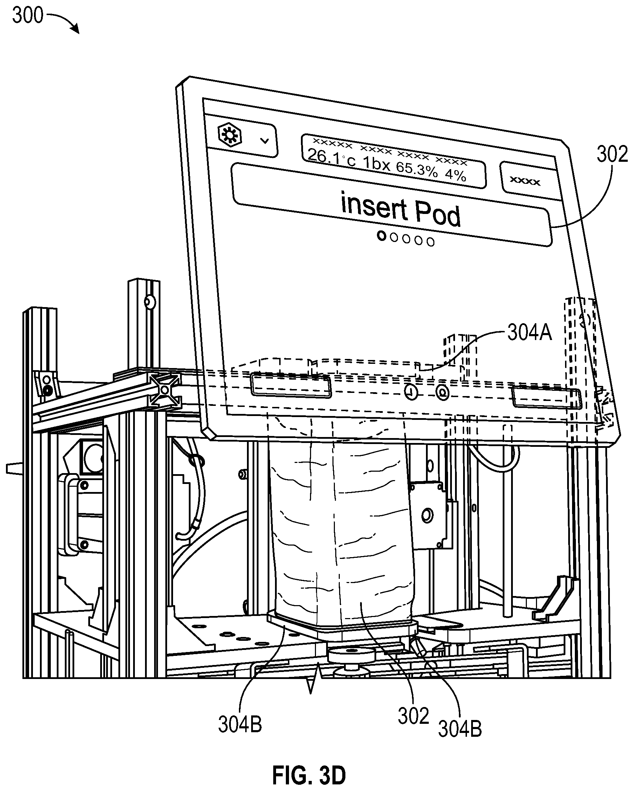

Figure 3B illustrates the dispenser 300 when a cell culture solution (e.g., cell culture media) powder container 308 is installed or inserted into the dispenser. Although the container 308 is described as a cell culture solution (e.g., cell culture media) powder container used with a cell culture dispenser, it is understood that the container 308 may be used for other applications. In some embodiments, the cell culture solution (e.g., cell culture media) powder container 308 is container 100 or container 150. As illustrated, the container 308 is in a collapsed configuration; however, the container 308 may be installed or inserted into the dispenser 300 in other configurations. In some embodiments, the container 308 is installed or inserted by using guides (e.g., guides 122, guides 172) of the container to slide the container into slots 304. When the container 308 is installed or fully inserted into the dispenser, the rigid ends (e.g., first end 102, second end 104, first end 152, second end 154) of the container 308 are at a fixed position relative to the slots 304. In some embodiments, additionally, the air and water outlets of the dispenser 300 align with the inlets 108A and 108B or 158A and 158B such that sliding in the container into the slots would cause the outlets of the dispenser to couple with the inlets of the container without additional adjustment to align the container with the dispenser. In some embodiments, the air and water outlets of the dispenser 300 provide air and water (e.g., USP grade water, water suitable for cell culture solution (e.g., cell culture media) manufacturing). In some embodiments, alignment and coupling between the air and water outlets of the dispenser 300 and the inlets 108A and 108B or 158A or 158B are performed subsequently (e.g., when the container 308 is in an expanded configuration, as illustrated in Figure 3D).

In some embodiments, upon installation or insertion of the container 308, the GUI 302 is updated to prompt a user to begin the cell culture manufacturing process. In some embodiments, the GUI 302 is updated to display a selectable option to begin the cell culture manufacturing process.

In some embodiments, the content of the container 308 is identified by the dispenser 300, and the cell culture manufacturing process is performed based on the identification of the content in the container 308. In some embodiments, the content is identified using a QR code (e.g., QR code 120, QR code 170) on the container 308. In some embodiments, the dispenser 300 includes a scanner or a camera configured to scan identifying information (e.g., QR code, barcode, patterned code, radio frequency identification) on the container to identify the container content. In some embodiments, the camera or a second camera of the dispenser 300 is configured to monitor the chamber of the dispenser; images or video from the camera may be viewed on the display of the dispenser 300 or on a display of a second device in communication with the dispenser 300. For example, a sterility of the chamber is monitored based on the camera, and based on the monitoring, in accordance with a determination that the chamber requires sterilization, the dispenser 300 performs a sterilization operation. As another example, the cell culture solution manufacturing is monitored using the camera, and based on the monitoring, the dispenser 300 adjusts a manufacturing parameter (e.g., stirrer speed, mixing time). In some embodiments, a second device (e.g., a mobile device) is used to scan the identifying information (e.g., the second device uses an application to scan the identify information and communicate the identifying information with the dispenser 300). In some embodiments, the GUI 302 presents information about the content in the container 308 identified by the dispenser 300. In some embodiments, the GUI 302 presents selectable or configurable options for a user to input or update information regarding the identified content.

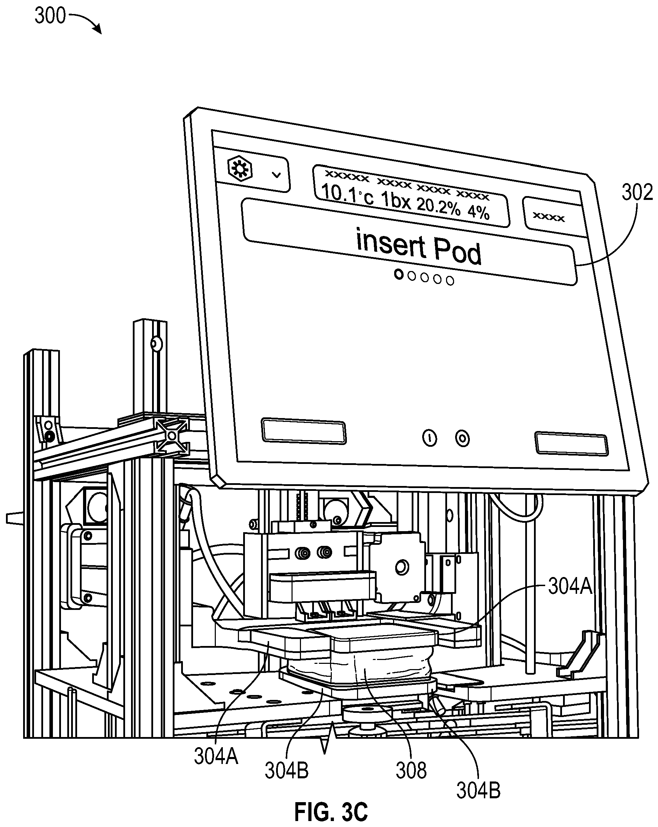

Figure 3C illustrates the dispenser 300 when the cell culture solution (e.g., cell culture media) powder container 308 is expanding. In some embodiments, the dispenser 300 expands the cell culture solution (e.g., cell culture media) powder container 308 in response to an input associated with a command to begin the cell culture solution (e.g., cell culture media) manufacturing process. In some embodiments, as described with respect to Figure 3B, the rigid ends (e.g., first end 102 or 152, second end 104 or 154) of the container are locked at a fixed position to a respective slot 304. More specifically, the rigid first end of the container 308 are locked at a fixed position to slots 304A, and the rigid second end of the container 308 are locked at a fixed position to slots 304B.

In some embodiments, when dispenser 300 expands the container 308, the dispenser causes the slots 304A and slots 304B to move away from each other in a direction parallel to a direction of the intended expansion (e.g., vertically). Because the rigid ends are locked at fixed positions to the respective slots, the middle portion (e.g., middle portion 106, middle portion 156) of the container 308 is caused to be expanded (e.g., toward an expanded configuration of the container, such as the configuration illustrated in Figure 1), and a volume in the container increases as the rigid ends move farther apart from each other.

In some embodiments, when the container 308 is being expanded by the dispenser 300, the GUI 302 presents a progress of the cell culture solution (e.g., cell culture media) manufacturing process. In some embodiments, the current step, the container content, the solution being manufactured, and a progress is shown on the GUI 302.

Figure 3D illustrates the dispenser 300 when the cell culture solution (e.g., cell culture media) powder container 308 is in an expanded configuration. In some embodiments, the expanded configuration of the cell culture solution (e.g., cell culture media) power container 308 is the expanded configuration of container 100 described with respect to Figure 1.

In some embodiments, compared to Figure 3C, the slots 304A and slots 304B are moving further away from each other, causing the volume in the container to be greater. In some embodiments, at this point shown in Figure 3D, the slots 304A and slots 304B (which are behind the GUI 302) stop moving away from each other. In some embodiments, a desired volume is reached by achieving a corresponding separation between the rigid ends of the container. In some embodiments, the volume of the container required to adequately mix a solution associated with the content in the container 308.

In some embodiments, when the desired volume of the container 308 is reached and the slots 304A and slots 304B stop expanding the container 308, the dispenser 300 aligns and couples the air and water outlets of the dispenser 300 and the inlets 108A and 108B or 158A or 158B.

In some embodiments, after the air and water outlets of the dispenser 300 and the inlets of the container 308 are aligned and coupled, mixing of the solution begins. In some embodiments, the air and water outlets of the dispenser 300 begin providing a suitable amount of air and water (e.g., USP grade water, water suitable for cell culture solution (e.g., cell culture media) manufacturing) into the container 308. In some embodiments, additionally, the dispenser 300 generates a magnetic field near the rigid second end (e.g., the rigid end coupled to slots 304B) of the container 308 to cause a stirrer included in the container to start rotating (e.g., clockwise relative to a surface of the rigid second end, counter-clockwise relative to a surface of the rigid second end).

In some embodiments, the content of the container is mixed using the stirrer for a pre-determined amount of time. In some embodiments, the amount of time is determined based on the cell culture solution (e.g., cell culture media) being manufactured. In some embodiments, identifying information (e.g., QR code, barcode, patterned code, radio frequency identification) on the container 308 includes information relating to mixing time, and the information relating to mixing time is input to the dispenser 300 when the content of the container is identified.

In some embodiments, when the solution is determined to be complete (e.g., the pre-determined amount of time has lapsed, the solubility of the liquid is determined to be achieved through measurement), the solution exits from an outlet (e.g., outlet 110, outlet 160) of the container 308 into the second container 306. In some embodiments, the solution passes through a filter, and the filter is included in the container 308. In some embodiments, prior to the solution exiting, the dispenser 300 opens a covering (e.g., a cap) of the second container 306 to allow the solution to be transferred from the container 308 to the second container 306. For example, the dispenser 300 includes a motor driven element that is configured to unscrew a cap of the second container 306 in accordance with a determination that the solution in the container 308 is ready for dispensing. In some embodiments, while the cap of the second container 306 is being removed, the second container is being lowered to separate the cap from the second container 306.

In some embodiments, after the solution is moved to the second container 306, the solution is suitable for the growth of cells or transferred to facilitate the growth of cells. Although the content of the container is described with respect to cell growth (e.g., cell culture solution (e.g., cell culture media)), it is understood that the description of the container content is not limited to cell growth. For example, cell growth may include tissue growth. As another example, cell culture may include tissue culture.

In some embodiments, the dispenser 300 performs the reverse steps (e.g., after a solution is created and is ready for dispensing in the second container 306). For example, the dispenser 300 includes a motor driven element that is configured to screw a cap of the second container 306 in accordance with a determination that the solution in the container 308 has been completely transferred. In some embodiments, while the cap of the second container 306 is being installed, the second container is being raised to mate the cap with the second container 306. In some embodiments, an interface between the dispenser 300 and the cap of the second container 306 is adjustable (e.g., the diameter of the interface is adjustable) to accommodate different container sizes.

In some embodiments, the dispenser 300 includes an enclosure or chamber (not shown). As an exemplary advantage, the enclosure allows for the sterile environment during preparation of the solution or when the solution is transferred from the cell culture solution (e.g., cell culture media) container. In some embodiments, the dispenser 300 comprises a heating chamber to heat water and air to sterilize the dispenser's piping. In some embodiments, the dispenser 300 comprises an ozone generator for removing contaminants in the chamber (e.g., to purify water, to purify air).

In some embodiments, when the container 308 is expanded to the expanded configuration and the solution begins to mix, the GUI 302 presents a progress of the cell culture solution (e.g., cell culture media) manufacturing process. In some embodiments, the current step, the container content, the solution being manufactured, and a progress is presented on the GUI 302.

In some embodiments, the container 308 is used in a bioreactor. Instead of a second container 306, as illustrated, the solution created from the contents of the container 308 is transferred to a bioprocessing vessel (e.g., the bioreactor provides a connection that allows fluid to flow from the container 306 to the bioprocessing vessel).

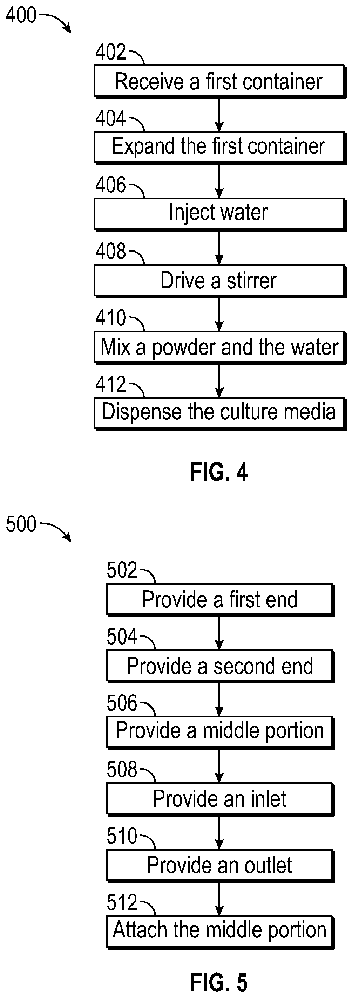

Figure 4 illustrates an exemplary method 400 of operating an exemplary dispenser. In some embodiments, the method 400 is a method of operating the container 100, the container 150, the container 308, or the container 608. In some embodiments, the method 400 is performed using the dispenser 300 or dispenser 600. Although the method 400 is illustrated as including the described steps, it is understood that different order of steps, additional steps, or fewer steps may be performed to operate the exemplary container without departing from the scope of the disclosure. In some embodiments, the steps of method 400 are performed in response to a selection of a GUI object on a GUI (e.g., GUI 302, GUI 602) of the dispenser.

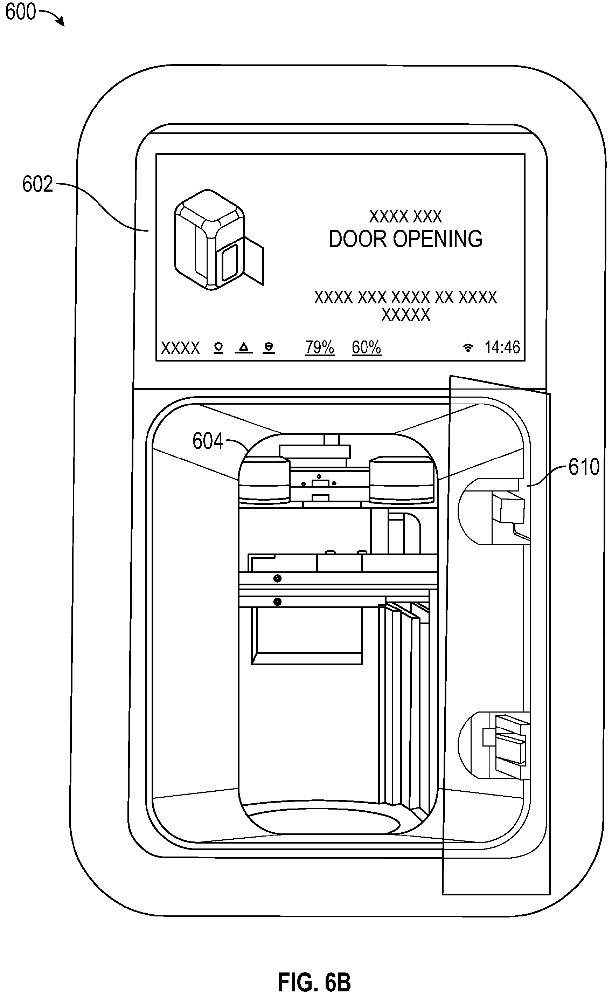

In some embodiments, the method 400 includes receiving a first container in a collapsed configuration into a dispenser (step 402). For example, the dispenser 300 receives the container 308 inserted into the dispenser, as described with respect to Figure 3B. In some embodiments, the first container is inserted after a door (e.g., door 610) of the dispenser opens, as described with respect to Figure 6B.

In some embodiments, the method 400 includes sterilizing the dispenser. For example, prior to insertion of the container 308 or prior to mixing, components (e.g., a chamber where the first container 308 is inserted, a chamber where the second container 306 is placed, an inlet of the dispenser 300) of the dispenser 300 are sterilized (e.g., by the dispenser 300, by a device different from the dispenser 300). In some embodiments, the components are sterilized using a sterilization light (e.g., a UV light). As another example, after a cell culture solution is created and retrieved, the dispenser performs the sterilization. In some embodiments, the dispenser comprises a heating chamber to heat water and air to sterilize the dispenser's piping. In some embodiments, the dispenser comprises an ozone generator for removing contaminants in the chamber (e.g., to purify water, to purify air).

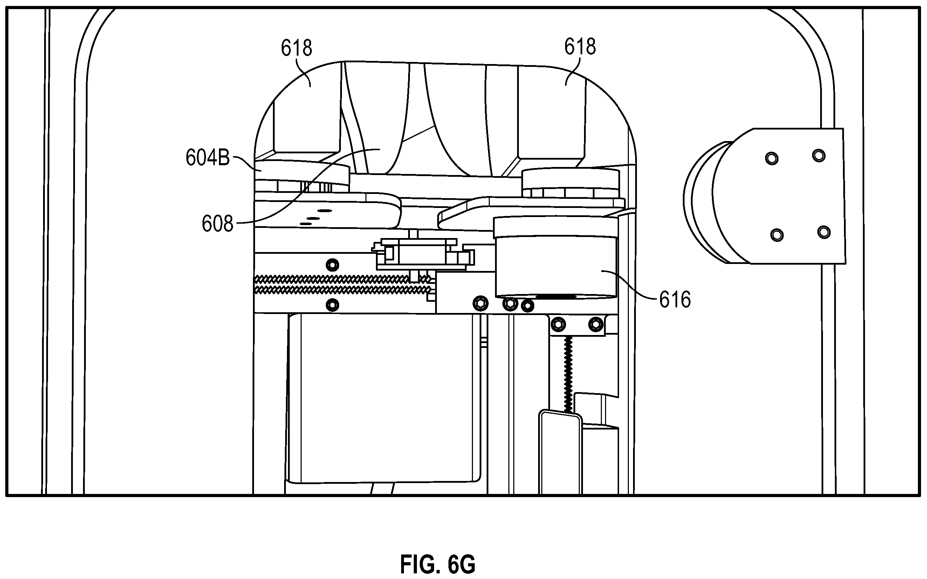

In some embodiments, the method 400 includes expanding the first container to an expanded configuration (step 404). For example, the dispenser 300 expands the container 308, as described with respect to Figure 3C. As another example, the dispenser 600 expands the container 608, as described with respect to Figure 6G.

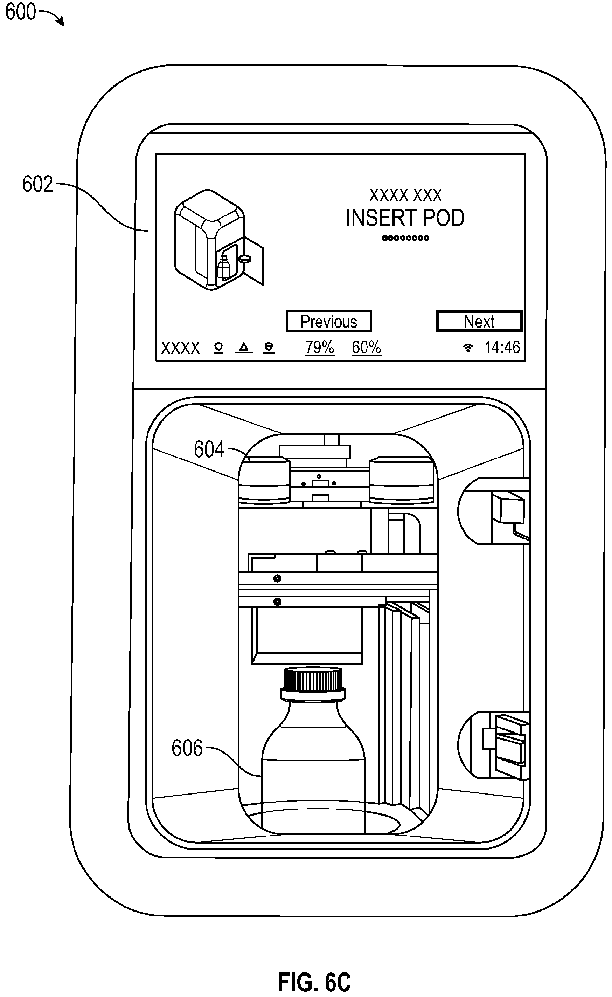

In some embodiments, the method 400 includes receiving an input. In some embodiments, the input is a selection on a GUI of the dispenser, and the first container is expanded in response to receiving the input. For example, a user provides an input by making a selection on the GUI 302, and in response to the selection, the dispenser 300 expands the container 308 and mixing begins. In some embodiments, the input is a QR code scan of a QR code of the first container, and the first container is expanded in response to receiving the input. For example, a QR code (e.g., QR code 120, QR code 170) of the container 308 is scanned (e.g., by the dispenser 300, by a different than the device 300), and in response to the scanning, the dispenser 300 expands the container 308 and mixing begins. As another example, as described with respect to Figure 6C, after the container 608 is inserted into the dispenser 600, the user selects GUI object 622C to proceed with the input for creating the cell culture solution.

In some embodiments, the method 400 includes while the first container is expanding or the first container is in the expanded configuration, injecting water into the first container (step 406). For example, while the dispenser 300 expands the container 308 or when the container 308 is in the expanded configuration, the dispenser 300 injects water (e.g., USP grade water) in the container 308, as described with respect to Figures 3C and 3D. In some embodiments, the method 400 includes while the first container is expanding or the first container is in the expanded configuration, injecting air into the first container. For example, while the dispenser 300 expands the container 308 or when the container 308 is in the expanded configuration, the dispenser 300 injects air in the container 308, as described with respect to Figures 3C and 3D. As another example, as described with respect to Figure 6H, water is injected into the container 608.

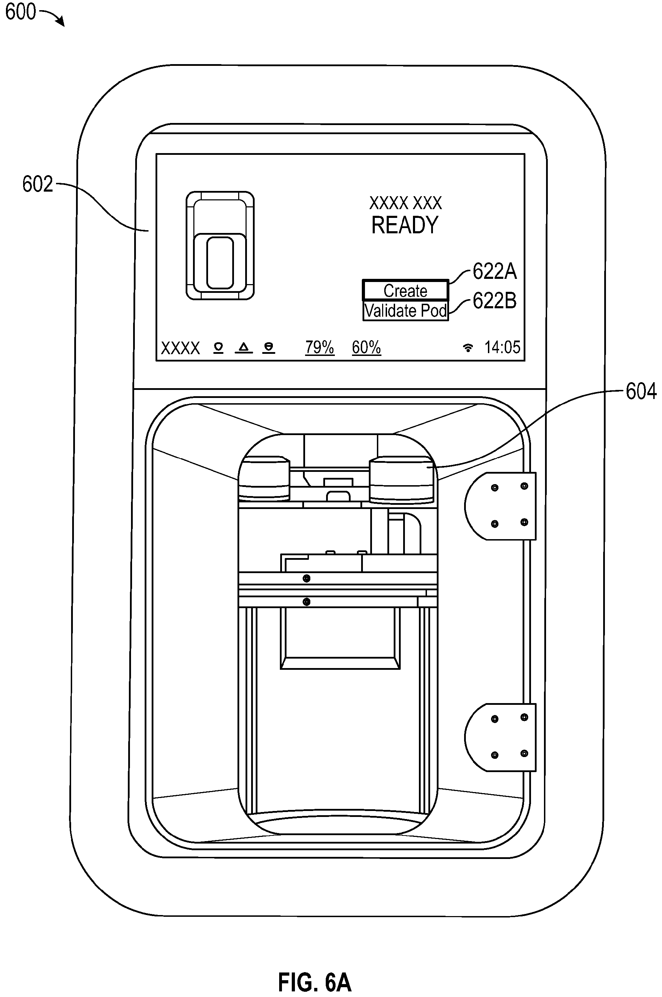

In some embodiments, the method 400 includes applying a baffle to the first container. In some embodiments, the baffle is applied to the first container while the first container is expanding or the first container is in the expanded configuration. For example, as described with respect to Figure 6H, a baffle is applied to the middle portion of the container 608 to support the middle portion of the container while cell culture media solution is being created. In some embodiments, the baffle is applied prior to the expansion of the first container.

In some embodiments, the method 400 includes while the first container is expanding or the first container is in the expanded configuration, driving a stirrer in the first container (step 408). For example, while the dispenser 300 expands the container 308 or when the container 308 is in the expanded configuration, the dispenser 300 drives a stirrer in the container 308, as described with respect to Figures 3C and 3D. In some embodiments, the stirrer is driven magnetically. For example, as described herein, the dispenser 300 provides a time varying magnetic field to control the movement of the stirrer to mix the powder and water. As an example, the stirrer is driven to rotate at 2000 RPM. As another example, as described with respect to Figure 6H, the magnetic field generator 616 magnetically drives the stirrer to mix the content of the container 608.

In some embodiments, the method 400 includes while the first container is expanding or the first container is in the expanded configuration, mixing, with the stirrer, a powder and the water in the first container to create the culture media (step 410). For example, while the dispenser 300 expands the container 308 or when the container 308 is in the expanded configuration, the stirrer in the container 308 mixes a powder (e.g., cell culture solution (e.g., cell culture media) powder) and the injected water, as described with respect to Figures 3C and 3D. As another example, as described with respect to Figure 6H, the magnetic field generator 616 magnetically drives the stirrer to mix the content of the container 608.

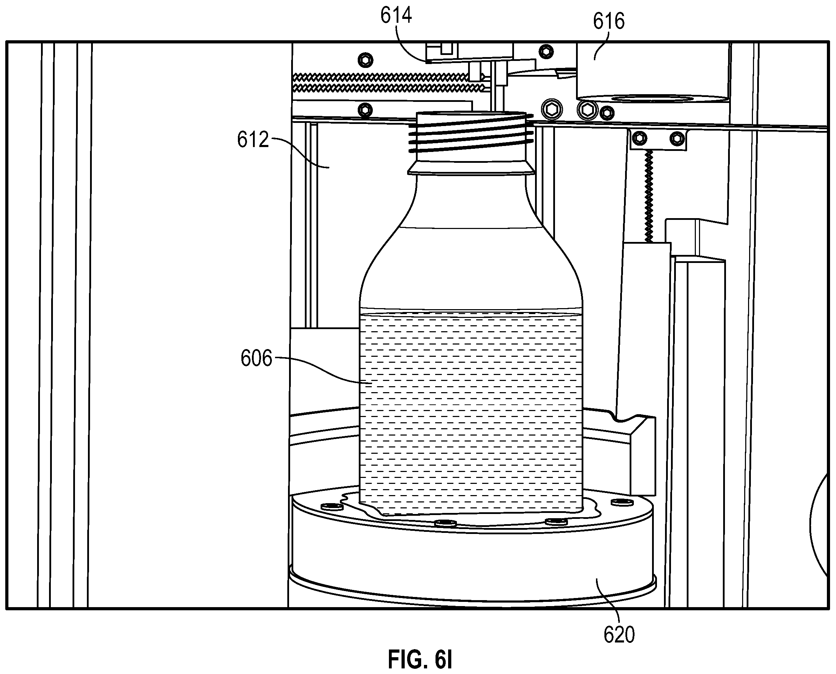

In some embodiments, the method 400 includes dispensing the culture media into a second container (step 412). For example, after the powder and the water finish mixing and culture media is created, the dispenser 300 dispenses the culture media into a second container 306 from the first container 308. As another example, as described with respect to Figure 6I, the dispenser 600 dispenses the cell culture solution into the second container 606 from the first container 608.

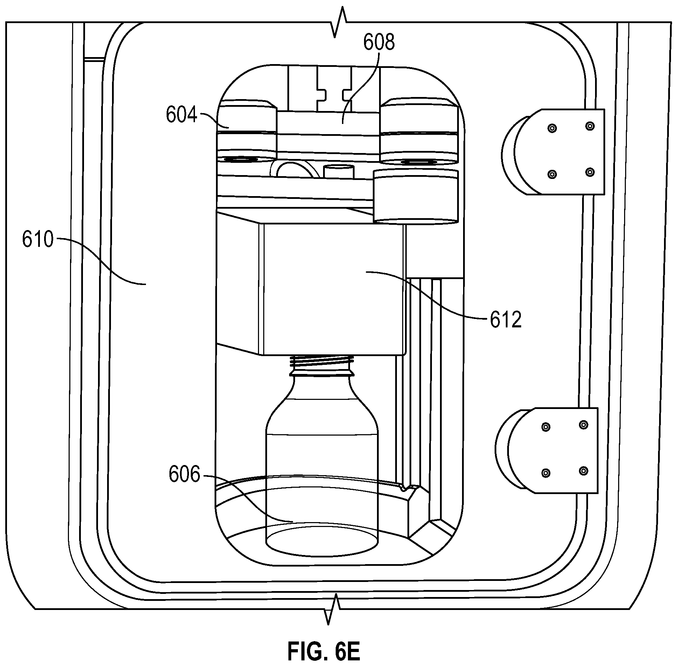

In some embodiments, the method 400 includes prior to dispensing the culture media into the second container, removing a cover of the second container. For example, prior to dispensing the culture media into the second container 306, the dispenser 300 unscrews a cap of the second container 306. As another example, as described with respect to Figure 6E, the second container access mechanism 612 unscrews a cap of the second container 606 prior to dispensing the culture media into the second container 606. In some embodiments, the method 400 includes providing a cover to the second container after dispensing the culture media into the second container. For example, after the culture media is dispensed into the second container 306, the dispenser screws in a cap of the second container 306.

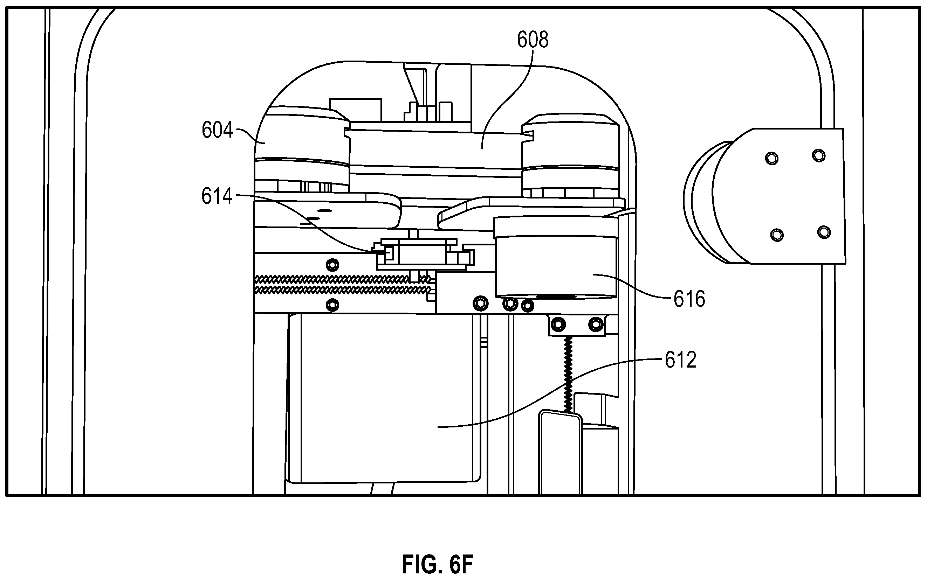

In some embodiments, the method 400 includes providing a filter. For example, prior to dispensing the culture media into the second container 306, a suitable filter, as described herein, is provided (e.g., by the dispenser 300). In some embodiments, the culture media is dispensed into the second container through the filter. For example, prior to dispensing the culture media into the second container 306, the filter removes undesired content (e.g., contaminate) from the solution in the first container 308 before the culture media is dispensed into the second container 306. As another example, as described with respect to Figure 6F, the filter coupling mechanism 614 provides a filter and couples the filter to the outlet of the container 608. In some embodiments, a filter is integrated with the outlet 110, advantageously reducing a need to separately attach the filter to the outlet. For example, as described with respect to Figure 1A, the filter is integrated with the outlet 110. In some embodiments, a dispenser does not include a filter coupling mechanism 614 because the filter is integrated with the outlet.

In some embodiments, the method 400 includes collapsing the first container from the expanded configuration to the collapsed configuration. For example, after the mixing the powder and the water and/or when the culture media is ready to be dispensed into the second container 306, the container 308 is collapsed from the expanded configuration (e.g., by the dispenser 300) to empty the content of the container. As another example, as described with respect to Figure 6I, after the cell culture solution is created, the dispenser 600 collapses the container 608 using slots 604.

In some embodiments, the method 400 includes performing inline testing of culture media. For example, prior to dispensing the culture media (e.g., into the second container 306, into the second container 606, into a bioreactor) or prior to allowing retrieval of the second container (e.g., the media is tested before the dispenser door opens for retrieval), the culture media is tested for quality, as described with respect to the description of dispenser 600**.**

In some embodiments, the method 400 includes refrigerating the cell culture solution. For example, prior to dispensing the culture media (e.g., into a bioreactor) or prior to allowing retrieval of the second container (e.g., the media is stored and refrigerated before the dispenser door opens for retrieval), the culture media is refrigerated at a temperature of - 10-4 degrees Celsius, as described with respect to the description of dispenser 600**.**

In some embodiments, the method 400 includes transporting the cell culture solution to a destination and while transporting the cell culture solution, heating the cell culture solution. In some embodiments, heating the cell culture solution comprises heating a tube transporting the cell culture solution to a same temperature as the destination. For example, as described with respect to the description of dispenser 600, while the cell culture solution is transported or guided to a destination via a tube, the tube is heated to a same temperature as the destination. As an example, the tube is heated to a same temperature as a coupled bioreactor (e.g., advantageously maintaining a condition of the cell culture solution prior to dispensing the cell culture solution into the bioreactor). In some embodiments, the tube is heated to a temperature of 30-40 degrees Celsius (e.g., 37 degrees Celsius).

Additional examples and exemplary advantages associated with method 400 are described with respect to Figures 1A-1B, 2, 3A-3D, and 6A-6K. For brevity, these examples and advantages would not be described again.

Figure 5 illustrates an exemplary method 500 of manufacturing an exemplary container. In some embodiments, the method 500 is a method of manufacturing the container 100, the container 150, the container 308, or the container 608. Although the method 500 is illustrated as including the described steps, it is understood that different order of steps, additional steps, or fewer steps may be performed to manufacture the exemplary container without departing from the scope of the disclosure.

In some embodiments, the method 500 includes providing a rigid first end (step 502). For example, the first end 102 is provided for manufacturing container 100. As another example, the first end 102 is provided for manufacturing container 150. In some embodiments, the first end is formed by at least one of stamping, molding, and 3-D printing. It is understood that described methods of forming the first end are not meant to be limiting.

In some embodiments, the first end is rectangular or circular. In some embodiments, the first end includes at least one of PLA and PCL. In some embodiments, the first end includes a recyclable material. In some embodiments, providing the first end includes forming a tongue or a groove (e.g., guides 122, guides 172) on the first end configured to, during insertion of the container into a dispenser (e.g., dispenser 300), couple the container to a complimentary groove or tongue on the dispenser. In some embodiments, the first end includes interfacing features (e.g., for stacking, as described with respect to Figure 1B). In some embodiments, the first end comprises a notch configured to mate with an opening of the second end and lock the container in the collapsed configuration (e.g., as described with respect to Figure 1B).

In some embodiments, the rigid first end comprises an opening. The opening may be advantageously used for other steps of method 500 before being covered by an access port (e.g., access port cover 124, access port cover 174). In some embodiments, the method 500 includes providing a cover configured to occlude the opening. For example, an access port cover (e.g., access port cover 124, access port cover 174) is provided to occlude the opening of the rigid first end.

In some embodiments, the method 500 includes locating a QR code associated with the cell culture solution (e.g., cell culture media) powder on the rigid first end. For example, QR code 120 is located on the access port cover 124, or QR code 170 is located on access port cover 174. It is understood that the illustrated locations of the QR code are not meant to be limiting. The QR code may be located on other parts of the container.

In some embodiments, the method 500 includes providing a rigid second end (step 504). For example, the rigid second end 104 is provided for manufacturing container 100. As another example, the rigid second end 154 is provided for manufacturing container 150. In some embodiments, the second end is formed by at least one of stamping, molding, and 3-D printing. It is understood that described methods of forming the second end are not meant to be limiting.

In some embodiments, the rigid second end comprises a tapered surface on an interior to the container (e.g., as described with respect to Figure 1A), the tapered surface tapering down and toward a center of the rigid second end. In some embodiments, the second end includes at least one of polyethylene, PLA, and PCL. In some embodiments, the second end includes a recyclable material. In some embodiments, providing the second end includes forming a tongue or a groove (e.g., guides 122, guides 172) on the second end configured to, during insertion of the container into a dispenser (e.g., dispenser 300), couple the container to complimentary groove or tongue on the dispenser. In some embodiments, the second end includes interfacing features (e.g., for stacking, as described with respect to Figure 1B).

In some embodiments, the method 500 includes providing a middle portion (step 506). For example, the middle portion 106 is provided for manufacturing container 100. As another example, the middle portion 156 is provided for manufacturing container 150. In some embodiments, the middle portion is deformable. In some embodiments, when the container is in an expanded configuration, the middle portion include a rectangular cross-section (e.g., as describe with respect Figure 1A) or a circular cross-section. In some embodiments, the middle portion is a polyethylene bag or a polypropylene bag. In some embodiments, the middle portion includes a recyclable material. In some embodiments, the middle portion comprises a flexible material.

In some embodiments, the method 500 includes providing an inlet (step 508). In some embodiments, the inlet is located on the first end. For example, the inlet 108A or inlet 108B is provided for manufacturing container 100. As another example, the inlet 158A or inlet 158B is provided for manufacturing container 150. In some embodiments, the method 500 includes fluidly coupling the inlet to a region (e.g., region 126, enclosed by the first end, the second end, and the middle portion) of the container. In some embodiments, the method 500 includes attaching an adapter to the inlet. In some embodiments, the adapter comprises at least one of a Luer Lock adapter and a PVT adapter.

In some embodiments, the method 500 includes providing a second inlet located on the rigid first end. For example, the inlet 108B or inlet 108A is provided for manufacturing container 100. As another example, the inlet 158B or inlet 158A is provided for manufacturing container 150. In some embodiments, the method 500 includes positioning the first and second inlets so that they are parallel. For example, the inlets 108A and 108B are located in a parallel configuration in container 100. As another example, the inlet 158A and 158B are located in a parallel configuration provided in container 150.

In some embodiments, the method 500 includes providing an outlet (step 510). In some embodiments, the outlet is located on the second end. For example, the outlet 110 is provided for manufacturing container 100. As another example, the outlet 160 is provided for manufacturing container 150. In some embodiments, the method 500 includes fluidly coupling the outlet to a region (e.g., region 126, enclosed by the first end, the second end, and the middle portion) of the container. In some embodiments, the method 500 includes mounting the outlet on a spring mechanism. In some embodiments, the method 500 includes attaching an evacuation tube to the outlet. In some embodiments, the evacuation tube includes at least one of a Luer Lock fitting and a sealing nozzle. In some embodiments, the evacuation tube is bendable or flexible.

In some embodiments, the method includes occluding the outlet with a material on the second end. The material occluding the outlet is configured to tear or break in response to an applied pressure (e.g., 5-10 psi) above a threshold pressure (e.g., 2-5 psi). In some embodiments, the material occluding the outlet is different than the material of the second end. In some embodiments, the material occluding the outlet has an area of 1cm².

In some embodiments, the method 500 includes attaching the middle portion to the first end and the second end (step 512). For example, the middle portion 106 is attached to the first end 102 and to the second end 104 for manufacturing container 100. As another example, the middle portion 156 is attached to the first end 102 and to the second end 154 for manufacturing container 150. In some embodiments, attaching the middle portion to the first end and the second end comprises gluing the middle portion to the first end and to the second end. As an exemplary advantage, plastic components included in the container are reduced. In some embodiments, the method 500 includes providing a seal between the rigid first end and the middle portion.

In some embodiments, the method 500 includes loading cell culture solution (e.g., cell culture media) powder in the region. In some embodiments, the cell culture solution (e.g., cell culture media) powder is loaded into the region (e.g., region 126) through an opening (e.g., to be covered by access port cover 124, to be covered by access port cover 174) of the container. In some embodiments, after the container content (e.g., cell culture solution (e.g., cell culture media) powder) is loaded through the opening, an access port cover (e.g., access port cover 124, access port cover 174) covers the opening. In some embodiments, the cell culture solution (e.g., cell culture media) powder is loaded into the region through an inlet of the container. In some embodiments, the cell culture solution powder is loaded in the region after attaching the deformable middle portion to the first end and second end. In some embodiments, the cell culture solution powder is loaded in the region prior to attaching the deformable middle portion to the first end and second end.

In some embodiments, loading cell culture solution (e.g., cell culture media) powder in the region includes providing a powder sachet, as described herein, comprising the cell culture solution (e.g., cell culture media) powder. In some embodiments, the method 500 includes attaching the powder sachet to the rigid first end.

In some embodiments, the method 500 includes pre-granulating the cell culture solution (e.g., cell culture media) powder by spray addition of a pH correcting agent. For example, prior to loading the cell culture solution (e.g., cell culture media) powder into the container, the cell culture solution (e.g., cell culture media) powder is pre-granulated by spray addition of a pH correcting agent to advantageously control a pH level of the cell culture solution (e.g., cell culture media) powder. In some embodiments, the method 500 includes providing a pH sensor sensing a pH level associated with the cell culture solution (e.g., cell culture media) powder (e.g., a pH sensor to sense the pH level of the cell culture solution (e.g., cell culture media) powder). In some embodiments, the method 500 includes providing a dissolution sensor (e.g., to monitor the solubility of container contents).

In some embodiments, the method 500 includes providing a stirrer. In some embodiments, the stirrer comprises PTFE. Additional exemplary stirrers are described herein, and for the sake of brevity, they are not described again. In some embodiments, the stirrer is loaded into the region through an opening (e.g., to be covered by access port cover 124, to be covered by access port cover 174) of the container. In some embodiments, after the stirrer is loaded through the opening, an access port cover (e.g., access port cover 124, access port cover 174) covers the opening. In some embodiments, the stirrer is loaded into the region through an inlet of the container.

In some embodiments, the method 500 includes providing a barrier in the middle portion separating the cell culture solution (e.g., cell culture media) powder from at least one of the rigid first end and the rigid second end. In some embodiments, providing the barrier further comprises folding the middle portion to create the barrier. For example, when the container is in the collapsed configuration, the middle portion (e.g., a foldable bag) is folded such that the folds of the middle portion serves as sealed barriers (e.g., the content is separated from at least one of the first end and the second end by the barrier) for the content in the barrier. In some embodiments, a barrier is provided, and the barrier is not a part of the middle portion, as described herein.

In some embodiments, the barrier of the middle portion is created when the container changes from the expanded configuration to the collapsed configuration (e.g., when the container content is being loaded, after the content is loaded, after a first content is loaded and before a second content is loaded). In some embodiments, as the container changes from the expanded configuration to the collapsed configuration, a tool (e.g., an off-center slit that the middle portion passes through) causes the middle portion to fold in a specific manner, such that the fold of the middle portion (e.g., the fold of a bag) creates the barrier.

In some embodiments, the method 500 includes creating a vacuum in the region. In some embodiments, creating the vacuum in the region comprises removing air from the region through an opening of the rigid first end (e.g., inserting a tube through the opening to remove air from the region 126). In some embodiments, after the vacuum is created, an access port cover (e.g., access port cover 124, access port cover 174) covers the opening and maintains the vacuum in the region. In some embodiments, the vacuum is created through an inlet of the container.

In some embodiments, the method 500 includes providing a filter having a pore size of 0.22-1 µm. In some embodiments, the method 500 includes attaching a filter to the outlet. For example, the filter is coupled to the outlet of the container (e.g., outlet 110, outlet 160) externally (e.g., as described with respect to FIGs. 3A-3D and 4). As another example, the filter is coupled to the outlet of the container (e.g., outlet 110, outlet 160) internally. For example, the filter is loaded into the region and coupled to the outlet. In some embodiments, the method 500 includes attaching a filter attached to an evacuation tube (e.g., an evacuation tube attached to the outlet).

Additional examples and exemplary advantages associated with method 500 are described with respect to Figures 1A-1B, 2, 3A-3D, and 6A-6K. For brevity, these examples and advantages would not be described again.

Figures 6A-6K illustrate an exemplary dispenser 600. In some embodiments, the exemplary dispenser 600 is dispenser 200 or dispenser 300. In some embodiments, the dispenser 600 is a cell culture solution (e.g., cell culture media) dispenser. Although the exemplary dispenser 600 is described with respect to a smaller container (e.g., 500 mL), it is understood that a larger container (e.g., 5 L, 500L, 2000 L) may be used (e.g., expanded, its content being mixed) in a similar manner without departing from the scope of the disclosure. For example, the larger container may be used to create a larger volume solution for a bioreactor. Additionally, it is understood that the steps of cell culture solution manufacturing with the dispenser may include more steps, fewer steps, or different order of steps than described with respect to Figures 6A-6K. In some embodiments, the disclosed operations of the dispenser 600 allows cell culture media to be quickly created (e.g., in 20 minutes) in a user friendly, precise, and sterile manner.

Figure 6A illustrates the dispenser 600 prior to installation of a cell culture solution (e.g., cell culture media) powder container (e.g., container 100, container 150). In some embodiments, the dispenser 602 includes a display configured to present a GUI 602, slots 604, door 610 (shown in later figures), second container access mechanism 612 (shown in later figures), filter coupling mechanism 614 (shown in later figures), magnetic field generator 616 (shown in later figures), baffle 618 (shown in later figures), second container handling mechanism 620 (shown in later figures), and a camera or a scanner (not shown). In some embodiments, the dispenser 600 is configured to manufacture a cell culture solution from container 608 and dispense cell culture solution into the second container 606, as described in more detail herein.

In some embodiments, the dispenser 600 includes at least one pump (not shown). In some embodiments, the dispenser 600 includes a pump for pumping air into the container 608 (e.g., during expansion the middle portion of the container 608). In some embodiments, the dispenser 600 includes a pump for pumping air out of the container 608 (e.g., during collapsing of the middle portion of the container 608). In some embodiments, the dispenser 600 includes a pump for cleaning and/or sterilizing the chamber after use. For example, the pump drains liquid in the chamber and/or pumps in water or a cleaning solution for cleaning and/or sterilizing the chamber.

In some embodiments, the dispenser 600 includes a first pump that is configured to apply a first pressure and is configured to apply a second pressure. In some embodiments, the first and second pressures are different. In some embodiments, the dispenser 600 includes a pump that is configured to apply variable pressures. In some embodiments, by including a pump or pumps that can apply different pressures, the dispenser 600 advantageously enables different tasks to be performed (e.g., different tasks that one pump applying one pressure would not be able to perform). For example, a pump applies a first pressure (e.g., 10-30 psi) for pumping air into the container 608. As another example, a pump applies a second pressure (e.g., less than 12 psi, not pressurized) for pumping air out of the container 608. As yet another example, a pump applies a third pressure (e.g., less than 12 psi, not pressurized) for cleaning and/or sterilizing the chamber after use.

In some embodiments, some components of dispenser 600 are the components described with respect to Figure 1, 2, or 3. For example, GUI 602 is GUI 302, slots 604 is slots 304, the second container 606 is second container 306, container 608 is container 308, container 100, or container 150, and the camera or the scanner is described with respect to Figure 3. In some embodiments, dispenser 600 includes the filtration system 202, the first handling system 204, and the second handling system 206, as described with respect to Figure 2. For the sake of brevity, some embodiments and advantages of these components are not described here.

In some embodiments, the GUI 602 indicates a state of the dispenser 600 and/or presents selectable inputs for a user to control the dispenser 600 (e.g., for manufacturing cell culture media). For example, as illustrated in Figure 6A, in some embodiments, the GUI 602 indicates that the dispenser 600 is ready to manufacture a cell culture solution and presents GUI object 622A. In response to selection of the GUI object 622A, the dispenser 600 begins the cell culture manufacturing process (e.g., as described with respect to Figure 4). In some embodiments, the GUI 602 indicates other states of the dispenser 600. For example, the dispenser 600 may be connected to a wireless network for communication with other devices (e.g., a server for providing information associated with a cell culture solution, a second device for controlling the dispenser 600 wirelessly), and GUI object 622B indicates a network status (e.g., connected to WiFi network). In some embodiments, the GUI 602 includes additional GUI objects to indicate temperature (e.g., temperature of the chamber, ambient temperature, selection of the GUI object allows a user to adjust the temperature of the chamber), progress of a current manufacturing operation (and GUI elements for controlling the current manufacturing operation), pH level (e.g., sensed by a pH sensor of the dispenser), and/or a media being manufactured.

In some embodiments, in response to receiving a selection of the GUI object 622A, the cell culture manufacturing process begins. For example, as illustrated in Figure 6B, in response to the selection the door 610 opens, and the GUI 602 indicates the door opening state.

In some embodiments, as illustrated in Figure 6C, after the door 610 opens, the GUI 602 updates to prompt a user to insert a container 608 (e.g., pod) into slots 604. In some embodiments, the container 608 is inserted into slots 604 as described with respect to slots 304 and Figures 3A-3D. Additionally, as illustrated, during this time, a user may place a second container 606 into the chamber of the dispenser 600.

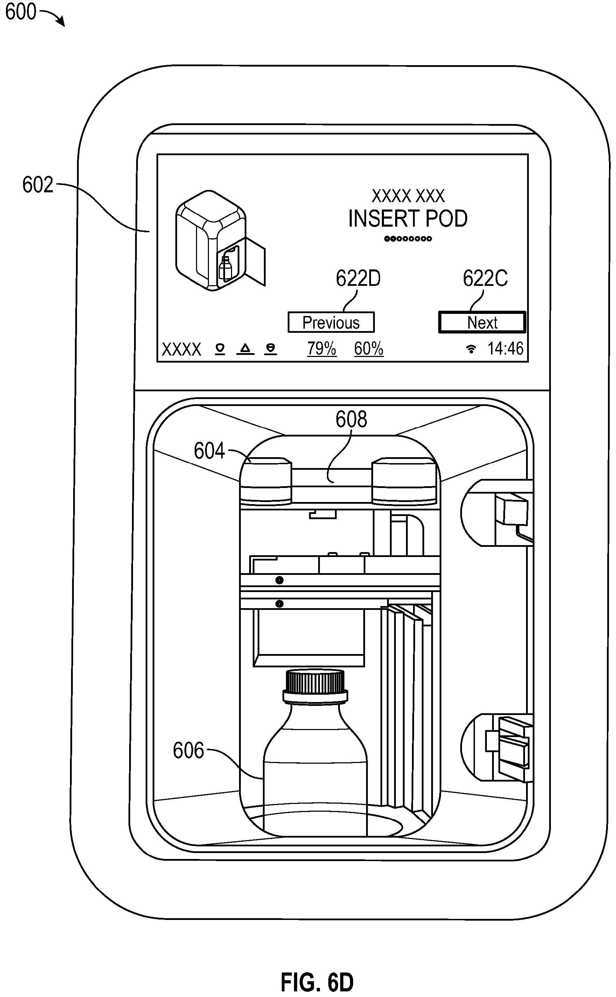

In some embodiments, as illustrated in Figure 6D, after the GUI 602 prompts the user to insert the container 608 into slots 604, a user may insert the container 608 (in a collapsed configuration) into the slots 604. In some embodiments, during this time, the GUI 602 includes GUI objects 622C and 622D. In response to selection of the GUI object 622C, the dispenser 600 proceeds to a next step of the cell culture manufacturing process. In some embodiments, if the container 608 is not inserted (or properly inserted) or the second container 606 is not placed (or not properly placed) into the chamber, the dispenser 600 does not respond to a selection of the GUI object 622C, or the GUI 602 does not display the GUI object 622C. A warning may be displayed on the GUI 602 to prompt a user to properly insert a container 608 or to properly place a second container 606. In response to selection of the GUI object 622D, the dispenser 600 may return to a previous page (e.g., door opening, a home page) on the GUI 602.

In some embodiments, in response to selection of the GUI object 622C, the dispenser 600 proceeds to a next step of the cell culture manufacturing process. In some embodiments, as illustrated in Figure 6E, in response to selection of the GUI object 622C, the door 610 closes, and the second container access mechanism 612 moves toward the door, such that the mechanism 612 is above the second container 606. In some embodiments, the second container access mechanism 612 is configured to open and close the second container 606, as described with respect to Figures 2 and 3A-3D. For the sake of brevity, some descriptions and advantages are not described here. After the second container access mechanism 612 is above and aligned to the second container 606, the second container access mechanism 612 opens the second container 606 (e.g., by unscrewing a cap of the second container 606). In some embodiments, after the second container 606 is opened, the second container access mechanism 612 retains the cap, while the cell culture solution is being manufactured. The second container access mechanism 612 may retain the cap until the cell culture solution is dispensed, and the mechanism 612 may screw the cap back onto the second container 606, as described in more detail herein. In some embodiments, in response to selection of the GUI object 622C, the GUI 602 updates to indicate that a current step of the cell culture manufacturing process is being performed. In some embodiments, the updated GUI 602 includes GUI objects for changing (e.g., stopping, updating manufacturing parameters) the cell culture manufacturing process being performed.

In some embodiments, as illustrated in Figure 6F, after the second container access mechanism 612 opens the second container 606, the mechanism 612 moves back to its initial location. After the second container 606 opens, the filter coupling mechanism 614 moves to align a filter (e.g., a filter as disclosed herein) with the outlet of the container 608 (e.g., outlet 110, outlet 160). After the filter is aligned with the outlet, the filter coupling mechanism 614 couples (e.g., attaches) the filter to the outlet. In some embodiments, a filter is integrated with the outlet, advantageously reducing a need to separately attach the filter to the outlet. For example, as described with respect to Figure 1A, the filter is integrated with the outlet 110. In some embodiments, the dispenser does not include the filter coupling mechanism 614 because the filter is integrated with the outlet.

In some embodiments, as illustrated in Figure 6G, the container 608 expands, as described with respect to Figures 3A-3D (e.g., slot 604B of slots 604 is slot 304B of slots 304). In some embodiments, while the container 608 expands, air and/or water (e.g., using a pump of the dispenser 600) is injected into the container 608 for cell culture media creation, as described with respect to Figures 3A-3D. In some embodiments, as illustrated in Figure 6H, the baffle 618 converges toward the container 608 in the expanded configuration. In this example, the baffle 618 converges toward the container 608 after the container 608 is in the expanded configuration so the baffle does not physically impede the container 608 from expanding. However, it is understood that the described timing of baffle application is not meant to be limiting. In some embodiments, the baffle does not physically impede container expansion, and the baffle converges prior to the container expanding.

In some embodiments, the structure of the baffle is concentric to the container 608 such that the structure supports the middle portion of the container while a force is acting on the middle portion (e.g., to prevent the middle portion for breaking). For example, as illustrated, the two components of the baffle 618 include planes that are parallel to the middle portion, optimizing points of contact between the middle portion and the baffle and hence, optimizing support for the middle portion.

It is understood that the described baffle geometry and mechanism are not meant to be limiting. The baffle may include different number of components (e.g., different than two) or different shapes than described, as long as the baffle is able to provide support for the middle portion of the container 608 when a force is acting on the middle portion. For example, the baffle may include four components (e.g., each component supporting a quarter of the middle portion). As another example, the shape of the baffle may be cylindrical, in a shape of a hexagonal prism, in a shape of an octagonal prism, and the likes.

In some embodiments, the baffle 618 advantageously provides support for the material of the middle portion (e.g., middle portion 106) of the container 608, while the container 608 is in the expanded configuration and a force acts on the middle portion (caused by, e.g., mixing of the cell culture solution, pressured applied (e.g., 5-10 psi) in the middle portion). By providing support for the middle portion of the container while a force acts on the middle portion, the baffle may allow a more compact (e.g., thinner) material to be used for manufacturing the middle portion. A more compact middle portion material may increase the efficiency of content storage and/or allow the ends of the container (e.g., the first end 102, the second end 104) to mate uniformly (e.g., allowing the container to close properly) when the container is in the collapsed configuration. For example, a thinner middle portion material allow more cell culture media powder to be stored and the container to be closed properly when the container is in the collapsed configuration.

In some embodiments, after the container 608 is in the expanded configuration, the magnetic field generator 616 (as described with respect to Figures 1A-1B, 2, and 3A-3D) moves to align with the second end of the container 618. After the magnetic field generator 616 aligns with the second end of the container 618 (e.g., when the magnetic field generator 616 is completely under the second end of the container 618), the magnetic field generator 616 generates a magnetic field, as described with respect to Figures 1A-1B, 2, and 3A-3D, to drive a stirrer in the container 608, causing the container content to mix.

In some embodiments, the magnetic field generator 616 continues to magnetically drive the stirrer until a desired cell culture solution is created. In some embodiments, the magnitude, direction, and/or frequency may change to drive the stirrer in a different manner while the cell culture solution is being created. In some embodiments, the solution may be stirred for a pre-determined amount of time (e.g., based on the container content). In some embodiments, the dispenser 600 includes a sensor (not shown) (e.g., a pH sensor, a timing sensor, an optical system for quantifying turbidity (e.g., in the solution)) for determining whether the desired cell culture solution has been created. In some embodiments the magnetic field generator 616 returns to its initial location after the desired cell culture solution has been created (e.g., stirring is completed).

In some embodiments, as illustrated in Figure 6I, the second container handling mechanism 620 raises the second container 608 toward the outlet of the container 606 and filter. In some embodiments, the second container handling mechanism 620 is configured to secure the second container 608 (e.g., keep the container from moving, keep the container from contacting contaminates) while the cell culture solution is being created (e.g., mixed).

In some embodiments, the second container handling mechanism 620 raises the second container 608 toward the container 606 in accordance with a determination that the desired cell culture solution has been created (e.g., after a pre-determined amount of time, the dispenser 600 determines that the solution has been created (e.g., based on sensor data)).

In some embodiments, after the second container 608 is raised at a suitable distance (e.g., a distance that allows the cell culture solution to be cleanly or efficiently dispensed) from the filter, the dispenser 600 causes the cell culture solution to be dispensed from the container 606. In some embodiments, the dispenser applies a pressure (e.g., 5-10 psi, using a pump fluidly coupled to the container, by collapsing the container 608 back to the collapsed configuration) in the middle portion of the expanded container to push the cell culture solution toward the outlet of the container 606. In some embodiments, the outlet is occluded by a material that is configured to tear or break in response to an applied pressure (e.g., 5-10 psi) above a pressure threshold (e.g., 2-5 psi). As such, in response to the pressure applied by the dispenser in the middle portion, the material occluding the outlet tears or breaks, allowing the cell culture solution to exit the outlet, pass through the filter coupled to the outlet, and be dispensed to the second container 608, as illustrated. In some embodiments, after the second container 608 is raised at the suitable distance from the filter, the dispenser 600 collapses the container 608 back to the collapsed configuration (e.g., the slots 604 moves towards each other to move the first and second ends of the container 608 closer to each other), pushing the cell culture solution out of the container 608 through the container's outlet.

In some embodiments, after the desired cell culture solution is dispensed into the second container 608, the second container handling mechanism 620 lowers the second container 608 back to its initial location. In some embodiments, after the desired cell culture solution is dispensed into the second container 608, the filter coupling mechanism 614 disposes the filter, as described with respect to Figures 1A-1B, 2, and 3A-3D. In some embodiments, a filter is integrated with the outlet, advantageously reducing a need to separately attach the filter to the outlet. For example, as described with respect to Figure 1A, the filter is integrated with the outlet 110. In some embodiments, a dispenser does not include a filter coupling mechanism 614 because the filter is integrated with the outlet; the filter is disposed with the second container 608.

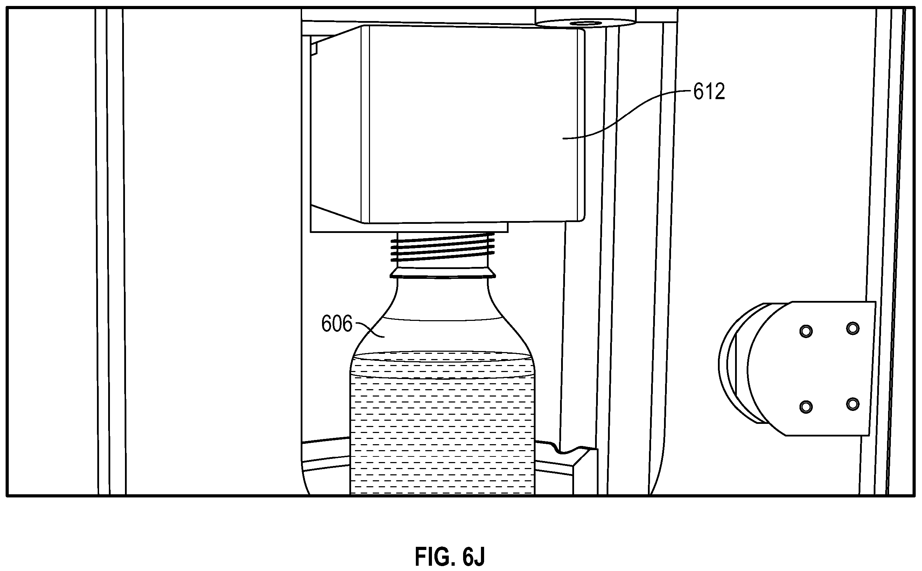

In some embodiments, as illustrated in Figure 6J, after the cell culture solution is dispensed into the second container 608 and the second container 608 is lowered, the second container access mechanism 612 moves to align with the second container 608. In some embodiments, the second container access mechanism 612 retained the cap of the second container 608 (e.g., after the mechanism 612 opens the cap). In some embodiments, after the second container access mechanism 612 aligns with the second container 608, the mechanism 612 closes the second container 608 (e.g., screws the cap back onto the second container 608).

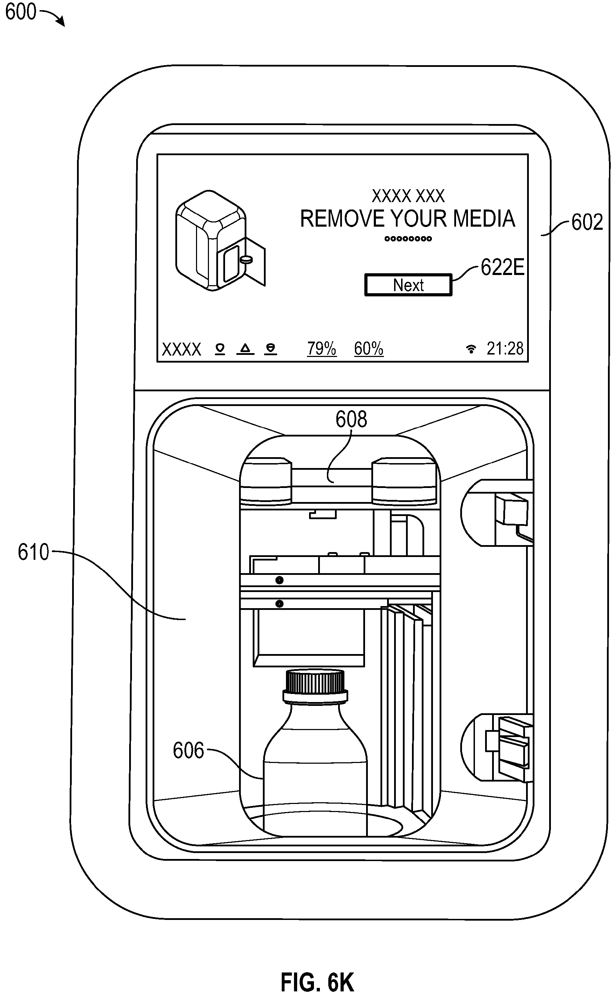

In some embodiments, as illustrated in Figure 6K, after the second container 608 is closed, the dispenser 600 causes the door 610 to open, allowing the user to retrieve the second container 608 and its content. In some embodiments, after the door 610 is open, the GUI 602 updates to prompt the user to retrieve the filled second container 608 and/or the container 606 (with its content emptied after the cell culture solution is created, for disposal or recycling).

In some embodiments, during this time, the GUI 602 includes a GUI object 622E. A user may select the GUI object 622E after the container 608 and second container 606 are retrieved (e.g., to inform the dispenser 600 of the retrieval of the containers). In response to selection of the GUI object 622E, the dispenser 600 closes the door 610. In some embodiments, in response to selection of the GUI object 622E, the GUI 602 updates to display the home page. In some embodiments, the GUI object 622E does not appear until the container 608 and second container 606 are removed from the dispenser 600 (e.g., based on sensors of the dispenser 600). In some embodiments, the GUI object 622E is not selectable until the container 608 and second container 606 are removed from the dispenser 600 (e.g., based on sensors of the dispenser 600).

In some embodiments, in response to the door 610 closing, the dispenser 600 prepares for manufacturing another cell culture solution (e.g., the dispenser 600 is sterilized, as described with respect to Figures 2 and 3A-3D).

In some embodiments, the dispenser 600 is configured for inline testing of cell culture solution. For example, the inline testing of media is performed to determine the quality of the media (e.g., amino acid levels, protein levels, salt levels, and the likes). In some embodiments, the inline testing of the media is performed by a sensor of the dispenser 600. In some embodiments, the dispenser 600 manufactures larger values of cell culture media (e.g., for a bioreactor). The inline testing advantageously confirms media suitability prior to dispensing the media into a bioreactor to prevent lower quality media from being dispensed into cells in the bioreactor (e.g., lower quality media may kill thousands or millions of dollars worth of cells in the bioreactor).

In some embodiments, the dispenser 600 is configured to refrigerate the cell culture solution. In some embodiments, the cell culture solution is refrigerated at a temperature of -10-4 degrees Celsius. For example, prior to retrieval of the cell culture solution (e.g., retrieval second container 606, retrieval of a container storing the cell culture solution, dispensing of the cell culture solution into a bioreactor), the chamber of the dispenser provides refrigeration (e.g., by lowering the temperature of the chamber) until the cell culture solution is ready for retrieval (e.g., retrieval second container 606, retrieval of a container storing the cell culture solution, dispensing of the cell culture solution into a bioreactor). In some embodiments, by refrigerating the cell culture solution, the dispenser advantageously allow the cell culture solution to be stored at ideal conditions and prevent cell culture solution degradation. In some embodiments, the cell culture solution is refrigerated at a different, thermally isolated chamber of the dispenser (e.g., different from a chamber for manufacturing cell culture solution), advantageously allowing a first cell culture solution to be stored and refrigerated in this chamber while a second cell culture solution is being manufactured in another chamber of the dispenser.

In some embodiments, the dispenser 600 comprises a tube configured to be heated or is configured to couple to a tube configured to be heated. In some embodiments, the tube is configured to transport or guide the cell culture solution to a destination (e.g., a bioreactor, a second container). In some embodiments, while the cell culture solution is transported or guided to the destination, the tube is heated to a same temperature as the destination. For example, the tube is heated to a same temperature as a coupled bioreactor (e.g., advantageously maintaining a condition of the cell culture solution prior to dispensing the cell culture solution into the bioreactor). In some embodiments, the tube is heated to a temperature of 30-40 degrees Celsius (e.g., 37 degrees Celsius).

Although the content of the container is described with respect to cell growth (e.g., cell culture solution (e.g., cell culture media)), it is understood that the description of the container content is not strictly limited to cell growth. For example, cell growth may include tissue growth. As another example, cell culture may include tissue culture.

In one aspect, a cell culture solution container comprises: a rigid first end; a rigid second end; a deformable middle portion attached to the first end and second end and enclosing, with the first and second ends, a region having a variable volume; cell culture solution powder in the region; an inlet fluidly coupled to the region; and an outlet fluidly coupled to the region, wherein the container comprises a collapsed configuration and an expanded configuration.

In some aspects of the above container, a volume of the region is greater in the expanded configuration than in the collapsed configuration.

In some aspects of the above containers, in the expanded configuration, the volume of the region accommodates a cell culture solution of 500 mL, 5 L, or 2000 L.

In some aspects of the above containers, a distance between the rigid first end and the rigid second end is greater in the expanded configuration than in the collapsed configuration.

In some aspects of the above containers, in the collapsed configuration, the distance is 0 mm, and in the expanded configuration, the distance is 120 mm or 300 mm.

In some aspects of the above containers, in the expanded configuration, the container has a height-to-width ratio of greater than one half.

In some aspects of the above containers, the ratio is 1.2.

In some aspects of the above containers, the inlet is located on the rigid first end, and the outlet is located on the rigid second end.

In some aspects of the above containers, the container further comprises a second inlet located on the rigid first end.

In some aspects of the above containers, the first and second inlets are parallel.

In some aspects of the above containers, the first inlet is a water inlet and the second inlet is an air inlet.

In some aspects of the above containers, the rigid first end is rectangular with a width of 85 mm and a length of 85 mm.

In some aspects of the above containers, the rigid first end is circular.

In some aspects of the above containers, when the container is in the expanded configuration, the middle portion comprises a rectangular cross-section.

In some aspects of the above containers, when the container is in the expanded configuration, the middle portion comprises a circular cross-section with a diameter of 73 mm.

In some aspects of the above containers, the rigid second end comprises a tapered surface on an interior to the container, the tapered surface tapering down and toward a center of the rigid second end.

In some aspects of the above containers, at least one of the rigid first end and the rigid second end is opaque to a sterilization light.

In some aspects of the above containers, the rigid first and second ends comprise at least one of polyethylene, PLA, and PCL.

In some aspects of the above containers, the middle portion is a polyethylene bag or a polypropylene bag.

In some aspects of the above containers, the rigid first and second ends and the middle portion comprise a recyclable material.

In some aspects of the above containers, the container further comprises an adapter attached to the inlet.

In some aspects of the above containers, the adapter comprises at least one of a Luer Lock adapter and a PVT adapter.

In some aspects of the above containers, the container further comprises a stirrer positioned in the region and having a length of 10-20 mm.

In some aspects of the above containers, the stirrer comprises PTFE.

In some aspects of the above containers, the container further comprises a QR code located on the rigid first end associated with the cell culture solution powder.

In some aspects of the above containers, the rigid first and second ends comprise a tongue or a groove configured to, during insertion of the container into a dispenser, couple the container and the dispenser.

In some aspects of the above containers, when the container is in the expanded configuration, the middle portion is flexible.

In some aspects of the above containers, a surface of the rigid first end is configured to interface with a surface of the rigid second end.

In some aspects of the above containers, wherein the middle portion stores a powder sachet, the powder sachet containing the solution powder.

In some aspects of the above containers, the powder sachet is attached to the rigid first end.

In some aspects of the above containers, the container further comprises a barrier in the middle portion separating the solution powder from at least one of the rigid first end and the rigid second end.

In some aspects of the above containers, the container further comprises a seal between the rigid second end and the middle portion.

In some aspects of the above containers, the container further comprises a seal between the rigid first end and the middle portion.

In some aspects of the above containers, the rigid first and second ends are configured to lock the container in the collapsed configuration when the rigid first end is rotated at a first angle from the rigid second end relative to an orientation of the rigid first and second ends when the container is in the expanded configuration.

In some aspects of the above containers, the rigid first end comprises a notch configured to mate with an opening of the rigid second end and lock the container in the collapsed configuration.

In some aspects of the above containers, the rigid first end comprises an opening.

In some aspects of the above containers, the container further comprises a cover configured to occlude the opening.

In some aspects of the above containers, the container further comprises a filter having a pore size of 0.22-1 µm.

In some aspects of the above containers, the container further comprises a filter attached to the outlet.

In some aspects of the above containers, the outlet is mounted on a spring mechanism.

In some aspects of the above containers, the outlet is configured to attach to an evacuation tube.

In some aspects of the above containers, the evacuation tube comprises at least one of a Luer Lock fitting and a sealing nozzle.

In some aspects of the above containers, the container further comprises a filter attached to the evacuation tube.

In some aspects of the above containers, the evacuation tube is bendable or flexible.

In some aspects of the above containers, the container further comprises a pH sensor sensing a pH level associated with the cell culture solution powder.

In some aspects of the above containers, the container further comprises a dissolution sensor.

In some aspects of the above containers, the cell culture solution powder is pre-granulated by spray addition of a pH correcting agent.

In some aspects of the above containers, in the collapsed configuration, the middle portion is configured to be vacuum-sealed.

In some aspects of the above containers, the container further comprises a material occluding the outlet, wherein the material is configured to break in response to an applied pressure above a pressure threshold.

In some aspects of the above containers, the material occluding the outlet has an area of 1 cm².

In some aspects of the above containers, the material comprises low-density polyethylene.

In some aspects of the above containers, the container further comprises a filter integrated with the outlet.

In one aspect, a method of creating culture solution comprises: receiving, at a dispenser, a first container in a collapsed configuration; expanding the first container to an expanded configuration; while the first container is expanding or the first container is in the expanded configuration: injecting water into the first container; driving a stirrer in the first container; and mixing, with the stirrer, a powder and the water in the first container to create the culture solution; and dispensing the culture solution into a second container.

In some aspects of the above method of creating culture solution, the first container is a cell culture solution container.

In some aspects of the above methods of creating culture solution, the method of creating culture solution further comprises receiving an input, wherein the input is a selection on a GUI of the dispenser, and the first container is expanded in response to receiving the input.

In some aspects of the above methods of creating culture solution, the method of creating culture solution further comprises receiving an input, and the input is a QR code scan of a QR code of the first container, and the first container is expanded in response to receiving the input.

In some aspects of the above methods of creating culture solution, the method of creating culture solution further comprises prior to dispensing the culture solution into the second container, removing a cover of the second container.

In some aspects of the above methods of creating culture solution, the method of creating culture solution further comprises providing a cover to the second container after dispensing the culture media into the second container.

In some aspects of the above methods of creating culture solution, the method of creating culture solution further comprises sterilizing the dispenser.

In some aspects of the above methods of creating culture solution, the method of creating culture solution further comprises injecting air into the first container.

In some aspects of the above methods of creating culture solution, the method of creating culture solution further comprises providing a filter, wherein the culture solution is dispensed into the second container through the filter.

In some aspects of the above methods of creating culture solution, the method of creating culture solution further comprises collapsing the first container from the expanded configuration to the collapsed configuration.

In some aspects of the above methods of creating culture solution, the stirrer is driven magnetically.

In some aspects of the above methods of creating culture solution, the first container is any of the above cell culture solution containers.

In some aspects of the above methods of creating culture solution, the method of creating culture solution further comprises applying a pressure to a middle portion of the first container.

In some aspects of the above methods of creating culture solution, the pressure is 5-12 psi.

In some aspects of the above methods of creating culture solution, the method of creating culture solution further comprises applying a second pressure to a chamber of the dispenser, the second pressure different from the first pressure.

In some aspects of the above methods of creating culture solution, the method of creating culture solution further comprises applying a baffle to support a middle portion of the first container.

In some aspects of the above methods of creating culture solution, the baffle is applied while the first container is expanding or the first container is in the expanded configuration.

In some aspects of the above methods of creating culture solution, the method of creating culture solution further comprises performing inline testing of the culture solution.

In some aspects of the above methods of creating culture solution, the method of creating culture solution further comprises refrigerating the culture solution at -10-4 degrees Celsius.

In some aspects of the above methods of creating culture solution, the method of creating culture solution further comprises: heating a tube coupled between the dispenser and a destination; and transporting, via the tube, the culture solution to a destination, wherein the tube is heated to a temperature of the destination at 30-40 degrees Celsius.

In one aspect, a cell culture solution dispenser is configured to any of the above methods of creating culture solution.

In one aspect, a method of manufacturing a cell culture solution container for storing culture solution powder comprises: providing a rigid first end; providing a rigid second end; providing a deformable middle portion; attaching the deformable middle portion to the first end and second end, the first end, second end, and middle portion enclosing a region having a variable volume; providing an inlet; fluidly coupling the inlet to the region; providing an outlet; fluidly coupling the outlet to the region; and loading cell culture solution powder in the region.

In some aspects of the above method of manufacturing, loading the culture solution powder into the region comprises loading the culture solution powder through an opening of the rigid first end.

In some aspects of the above methods of manufacturing, the method of manufacturing further comprises creating a vacuum in the region.

In some aspects of the above methods of manufacturing, creating the vacuum in the region comprises removing air from the region through an opening of the rigid first end.

In some aspects of the above methods of manufacturing, the method of manufacturing further comprises providing a stirrer.

In some aspects of the above methods of manufacturing, the stirrer comprises PTFE.

In some aspects of the above methods of manufacturing, the method of manufacturing further comprises providing a barrier in the middle portion separating the cell culture solution powder from at least one of the rigid first end and the rigid second end.

In some aspects of the above methods of manufacturing, providing the barrier further comprises folding the middle portion to create the barrier.

In some aspects of the above methods of manufacturing, the rigid first end comprises an opening.

In some aspects of the above methods of manufacturing, the method of manufacturing further comprises providing a cover configured to occlude the opening.

In some aspects of the above methods of manufacturing, providing the inlet comprises locating the inlet on the rigid first end, and providing the outlet comprises locating the outlet on the rigid second end.

In some aspects of the above methods of manufacturing, the method of manufacturing further comprises providing a second inlet located on the rigid first end.

In some aspects of the above methods of manufacturing, the method of manufacturing further comprises positioning the first and second inlets so that they are parallel.

In some aspects of the above methods of manufacturing, the rigid first end is rectangular.

In some aspects of the above methods of manufacturing, the rigid first end is circular.

In some aspects of the above methods of manufacturing, when the container is in an expanded configuration, the middle portion comprises a rectangular cross-section.

In some aspects of the above methods of manufacturing, when the container is an expanded configuration, the middle portion comprises a circular cross-section.

In some aspects of the above methods of manufacturing, the rigid second end comprises a tapered surface on an interior to the container, the tapered surface tapering down and toward a center of the rigid second end.

In some aspects of the above methods of manufacturing, the rigid first and second ends comprise at least one of polyethylene, PLA, and PCL.

In some aspects of the above methods of manufacturing, the middle portion is a polyethylene bag or a polypropylene bag.

In some aspects of the above methods of manufacturing, the rigid first and second ends and the middle portion comprise a recyclable material.

In some aspects of the above methods of manufacturing, the method of manufacturing further comprises attaching an adapter to the inlet.

In some aspects of the above methods of manufacturing, the adapter comprises at least one of a Luer Lock adapter and a PVT adapter.

In some aspects of the above methods of manufacturing, the method of manufacturing further comprises locating a QR code associated with the cell culture solution powder on the rigid first end.

In some aspects of the above methods of manufacturing, providing the rigid first and second ends comprises forming a tongue or a groove on the rigid first and second ends configured to, during insertion of the container into a dispenser, couple the container and the dispenser.

In some aspects of the above methods of manufacturing, the middle portion comprises a flexible material.

In some aspects of the above methods of manufacturing, the rigid first end and rigid second end comprises interfacing features.

In some aspects of the above methods of manufacturing, loading cell culture solution powder in the region further comprises providing a powder sachet comprising the cell culture solution powder.

In some aspects of the above methods of manufacturing, the method of manufacturing further comprises attaching the powder sachet to the rigid first end.

In some aspects of the above methods of manufacturing, the method of manufacturing further comprises providing a seal between the rigid first end and the middle portion.

In some aspects of the above methods of manufacturing, the rigid first end comprises a notch configured to mate with an opening of the rigid second end and lock the container in the collapsed configuration.

In some aspects of the above methods of manufacturing, the method of manufacturing further comprises providing a filter having a pore size of 0.22-1 µm.

In some aspects of the above methods of manufacturing, the method of manufacturing further comprises attaching a filter to the outlet.

In some aspects of the above methods of manufacturing, the method of manufacturing further comprises mounting the outlet on a spring mechanism.

In some aspects of the above methods of manufacturing, the method of manufacturing further comprises attaching an evacuation tube to the outlet.

In some aspects of the above methods of manufacturing, the evacuation tube comprises at least one of a Luer Lock fitting and a sealing nozzle.

In some aspects of the above methods of manufacturing, the method of manufacturing further comprises attaching a filter attached to the evacuation tube.

In some aspects of the above methods of manufacturing, the evacuation tube is bendable or flexible.

In some aspects of the above methods of manufacturing, the method of manufacturing further comprises providing a pH sensor sensing a pH level associated with the cell culture solution powder.

In some aspects of the above methods of manufacturing, the method of manufacturing further comprises providing a dissolution sensor.

In some aspects of the above methods of manufacturing, the method of manufacturing further comprises pre-granulating the cell culture solution powder by spray addition of a pH correcting agent.

In some aspects of the above methods of manufacturing, the cell culture solution powder is loaded in the region prior to attaching the deformable middle portion to the first end and second end.

In some aspects of the above methods of manufacturing, the container is any of the above cell culture solution container.

In some aspects of the above methods of manufacturing, the container is a container of any of the above methods of creating culture solution.

In some aspects of the above methods of manufacturing, the method of manufacturing further comprises occluding the outlet with a material, wherein the material is configured to break in response to an applied pressure above a pressure threshold.

In some aspects of the above methods of manufacturing, the material occluding the outlet has an area of 1 cm².

In some aspects of the above methods of manufacturing, the material comprises low-density polyethylene.

In one aspect, a dispenser for manufacturing a cell culture solution, comprising: a graphical user interface (GUI) for controlling the dispenser; slots for receiving a first container and expanding the first container; a second container access mechanism for opening and closing a second container, wherein the dispenser dispenses the cell culture solution to the second container; a second container handling mechanism for moving the second container toward and away a dispensing location of the cell culture solution; a filter providing mechanism for providing a filter to the first container; a magnetic field generator for magnetically driving a stirrer in the first container; and a baffle for supporting a middle portion of the first container.

In some aspects of the above dispenser, the dispenser further comprises a pump for applying a first pressure to a middle portion of the first container.

In some aspects of the above dispenser, the first pressure is 5-12 psi.

In some aspects of the above dispenser, the pump is further for applying a second pressure to a chamber of the dispenser, and the first pressure is different from the second pressure.

In some aspects of the above dispenser, the dispenser further comprises a second pump for applying a second pressure to a chamber of the dispenser.

In some aspects of the above dispenser, the baffle is for supporting a middle portion of the first container.

In some aspects of the above dispenser, the baffle is applied while the first container is expanding or the first container is in the expanded configuration.

In some aspects of the above dispenser, the dispenser is configured to perform inline testing of the cell culture solution.

In some aspects of the above dispenser, the dispenser is configured to refrigerate the culture culture solution at -10-4 degrees Celsius.

In some aspects of the above dispenser, the dispenser is configured to couple to a tube between the dispenser and a destination; and heat the tube to a temperature of the destination at 30-40 degrees Celsius.

In one aspect, a method of creating culture solution comprises: expanding a first container to an expanded configuration; while the first container is expanding or the first container is in the expanded configuration, mixing, a powder and water in the first container to create the culture solution; and dispensing the culture solution.

In some aspects of the above method of creating culture solution, the method of creating culture solution further comprises applying a pressure to a middle portion of the first container.

In some aspects of the above method of creating culture solution, the pressure is 5-12 psi.

In some aspects of the above method of creating culture solution, the method of creating culture solution further comprises applying a second pressure to a chamber of the dispenser, the second pressure different from the first pressure.

In some aspects of the above method of creating culture solution, the method of creating culture solution further comprises applying a baffle to support a middle portion of the first container.

In some aspects of the above method of creating culture solution, the baffle is applied while the first container is expanding or the first container is in the expanded configuration.

In some aspects of the above method of creating culture solution, the method of creating culture solution further comprises performing inline testing of the culture solution.

In some aspects of the above method of creating culture solution, the method of creating culture solution further comprises refrigerating the culture solution at -10-4 degrees Celsius.

In some aspects of the above method of creating culture solution, the method of creating culture solution further comprises: heating a tube coupled between the dispenser and a destination; and transporting, via the tube, the culture solution to a destination, wherein the tube is heated to a temperature of the destination at 30-40 degrees Celsius.

Although the disclosed embodiments have been fully described with reference to the accompanying drawings, it is to be noted that various changes and modifications will become apparent to those skilled in the art. Such changes and modifications are to be understood as being included within the scope of the disclosed embodiments as defined by the appended claims.

The terminology used in the description of the various described embodiments herein is for the purpose of describing particular embodiments only and is not intended to be limiting. As used in the description of the various described embodiments and the appended claims, the singular forms "a", "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

To preserve all the subject matter of the parent application in the present divisional application, the subject matter of the claims of the parent application is reproduced below as numbered clauses. The present application also discloses:
Clause 1:A cell culture solution container, comprising:
   a rigid first end;
   a rigid second end;
   a deformable middle portion attached to the first end and second end and enclosing, with the first and second ends, a region having a variable volume;
   cell culture solution powder in the region;
   an inlet fluidly coupled to the region; and
   an outlet fluidly coupled to the region, wherein:
      the container comprises a collapsed configuration and an expanded configuration.
Clause 2:The container of clause 1, wherein the rigid second end comprises a tapered surface on an interior to the container, the tapered surface tapering down and toward a center of the rigid second end.
Clause 3:The container of any of clause 1 or 2, wherein the middle portion is a polyethylene bag or a polypropylene bag.
Clause 4:The container of any of clauses 1-3, wherein the rigid first and second ends and the middle portion comprise a recyclable material.
Clause 5:The container of any of clauses 1-4, further comprising a stirrer positioned in the region.
Clause 6:The container of any of clauses 1-5, wherein the rigid first and second ends comprise a tongue or a groove configured to, during insertion of the container into a dispenser, couple the container and the dispenser.
Clause 7:The container of any of clauses 1-6, further comprising a filter attached to the outlet.
Clause 8:The container of any of clauses 1-7, wherein:
   the first inlet and a second inlet are located on the rigid first end, and
   the outlet is located on the rigid second end.
Clause 9:The container of any of clauses 1-8, wherein the rigid first and second ends comprise at least one of polyethylene, poly lactic acid (PLA), and poly carpolactone (PCL).
Clause 10:The container of any of clauses 1-9, wherein when the container is in the expanded configuration, the middle portion is flexible.
Clause 11:The container of any of clauses 1-10, wherein, in the collapsed configuration, the middle portion is configured to be vacuum-sealed.
Clause 12:The container of any of clauses 1-11, wherein at least one of the rigid first end and the rigid second end is opaque to a sterilization light.
Clause 13:A method of manufacturing a cell culture solution container of any of clauses 1-12, comprising:
   providing a rigid first end;
   providing a rigid second end;
   providing a deformable middle portion;
   attaching the deformable middle portion to the first end and second end, wherein the first end, second end, and middle portion enclosing a region having a variable volume;
   providing an inlet;
   fluidly coupling the inlet to the region;
   providing an outlet;
   fluidly coupling the outlet to the region; and
   loading cell culture solution powder in the region.
Clause 14:A method of creating culture solution, comprising:
   receiving, at a dispenser, a first container of any of clauses 1-12 in a collapsed configuration;
   expanding the first container to an expanded configuration;
   while the first container is expanding or the first container is in the expanded configuration:
      injecting water into the first container;
      driving a stirrer in the first container; and
      mixing, with the stirrer, a powder and the water in the first container to create the culture solution; and
      dispensing the culture solution into a second container.
Clause 15:A dispenser for manufacturing a cell culture solution, comprising:
   a graphical user interface (GUI) for controlling the dispenser;
   slots for receiving a first container of any of clauses 1-12 and expanding the first container;
   a second container access mechanism for opening and closing a second container, wherein the dispenser dispenses the cell culture solution to the second container;
   a second container handling mechanism for moving the second container toward and away a dispensing location of the cell culture solution;
   a filter providing mechanism for providing a filter to the first container;
   a magnetic field generator for magnetically driving a stirrer in the first container; and
   a baffle for supporting a middle portion of the first container.

## Claims

1. A system comprising:
a dispenser for manufacturing a cell culture solution,
a sensor configured to perform inline testing of the cell culture solution, and
a connection for transferring the cell culture solution from the dispenser to a bioreactor, the dispenser comprising:
an insertion mechanism comprising two portions configured to move apart,
wherein the insertion mechanism is configured to:
accept a container, the container comprising:
a first end,
a second end,
a middle portion,
a region having a variable volume, the first end, the second end, and the middle portion enclosing the region, and
cell culture solution powder in the region, wherein the container comprises a collapsed configuration and an expanded configuration;
after accepting the container, secure the container to the two portions; and
move the two portions apart to cause the container to expand to the expanded configuration.

2. The system of claim 1, further comprising a further sensor configured to perform inline testing of the cell culture solution, wherein the sensor is a pH sensor and the further sensor is an optical system for quantifying turbidity of the cell culture solution.

3. The system of claim 1 or 2, wherein the connection comprises a tube.

4. The system of any of claims 1-3, wherein the container further comprises a QR code associated with the cell culture solution powder.

5. The system of any of claims 1-4, wherein in the expanded configuration, the container accommodates 500 L of the cell culture solution.

6. The system of any of claims 1-5, wherein the first end is a first rigid end, and the second end is a second rigid end.

7. The system of any of claims 1-6, further comprising a filter attached to an outlet of the container, wherein the cell culture solution passes through the filter.

8. The system of any of claims 1-7, wherein the middle portion is a deformable middle portion.

9. The system of any of claims 1-8, wherein the middle portion is a polyethylene bag, a polypropylene bag, or another sterilizable plastic bag.

10. The system of any of claims 1-9, further comprising a stirrer positioned in the region.

11. The system of any of claims 1-10, wherein the container further comprises a tongue or a groove configured to, during insertion of the container into the dispenser, couple the container and the dispenser.

12. The system of any of claims 1-11, wherein the container further comprises an inlet fluidly coupled to the region and an outlet fluidly coupled to the region.

13. The system of any of claims 1-12, wherein the container comprises a recyclable material and/or the dispenser further comprises a graphical user interface, GUI, for controlling the dispenser.

14. The system of any of claims 1-13, wherein the dispenser is configured to inject water into the container and to mix the cell culture solution powder and the water in the container to create the cell culture solution.

15. A method of creating a cell culture solution, comprising:
receiving, at the dispenser of the system of any of claims 1-14, the container of the system of any of claims 1-14 in a collapsed configuration;
expanding the container to an expanded configuration;
while the container is expanding or the container is in the expanded configuration:
injecting water into the container;
mixing a cell culture solution powder and the water in the container to create the cell culture solution; and
transferring the cell culture solution to a bioreactor.
